# EUROPEAN PATENT APPLICATION

(11) **EP 3 632 913 A1**
(43) Date of publication of application: **08.04.2020**
(21) Application number: 18759357.9
(22) Date of filing: 31.05.2018
(51) Int. Cl.: C07D 471/04, A61P 17/06, A61K 31/517

(54) **CARBOXYLIC ACID DERIVATIVES OF PYRIDOQUINAZOLINES USEFUL AS PROTEIN KINASE INHIBITORS**

(30) Priority: 01.06.2017 ES 201730760
(71) Applicant: Oncostellae, S.L., 15705 Santiago de Compostela (ES)
(72) Inventor: KURZ, Guido, 08950 Esplugues Llobregat-Barcelona (ES); CAMACHO GÓMEZ, Juan, 31001 Pamplona-Navarra (ES)
(74) Representative: ABG Intellectual Property Law, S.L.
(86) International application number: PCT/ES2018/070397
(87) International publication number: WO 2018/220253

(57) **Abstract**

The present invention relates to novel carboxylic acid derivatives of 11-oxo-11H-pyrido [2,1-b]quinazoline-6-carboxamide as potent inhibitors of protein kinase, to pharmaceutical compositions containing them and to the use of said compounds for the manufacture of a medicament for the treatment of pathological conditions or diseases which can be improved by the inhibition of protein kinase

## Description

### Field of the Invention

The present invention relates to novel carboxylic acid derivatives conveniently substituted, as potent inhibitors of at least one protein kinase belonging to the family Mixed-lineage kinase (MLK), especially of Mixed-lineage kinase 3 (MLK3) or to the family of Janus kinases especially JAK3 and TYK2.

Other objectives of the present invention are to provide a procedure for preparing these compounds; pharmaceutical compositions comprising an effective amount of these compounds; the use of the compounds for manufacturing a medicament for the treatment of pathological conditions or diseases that can improve by inhibition of enzymes MLK, particularly MLK3 and Janus kinases selected from JAK3 and TYK2, such as liver diseases including non-alcoholic steatohepatitis (NASH) and cirrhosis of the liver, autoimmune diseases including psoriasis, atopic dermatitis, rheumatoid arthritis, multiple sclerosis, lupus, alopecia areata, inflammatory bowel diseases including ulcerative colitis and Crohn's disease, cancer such as gastric cancer, lung cancer, pancreatic cancer, breast cancer, colon cancer, colorectal cancer, ovarian cancer, prostate cancer and other solid tumours, melanoma, metastasizing cancers, cancer cachexia, and other diseases as asthma, chronic obstructive pulmonary disease (COPD), transplant rejection, haematological diseases, uveitis, dry eye and allergic conjunctivitis and neurodegenerative diseases including Alzheimer disease, among others.

### State of the art

Protein kinases are enzymes that play key regulatory roles in nearly every aspect of cell biology. These enzymes participate in signal transduction modules that regulate apoptosis, cell cycle progression, cytoskeletal rearrangement, differentiation, development, the immune response, nervous system function and transcription. Protein kinases represent attractive drug targets because their dysregulation occurs in a variety of illnesses including cancer, diabetes autoimmune, cardiovascular, inflammatory and nervous disorders. (Roskoski, R., Classification of small molecule protein kinase inhibitors based upon the structures of their drug-enzyme complexes, Pharmacological Research 103 (2016) 26-48).

The Mixed lineage kinases (MLKs) belong to a large group of Mitogen-activated protein kinase kinase kinases (MAP3Ks) that form part of the 3-tiered MAP3K-MAP2K-MAPK signalling pathways that relay a wide range of extracellular signals from the cell surface to the nucleus, thereby modulating gene expression. The mammalian MLK subfamily, which consists of the 4 members MLK1 (MAP3K9), MLK2 (MAP3K10), MLK3 (MAP3K11) and MLK4, activates well characterized mammalian MAPK pathways like ERK, JNK and p38 that regulate numerous cellular responses such as proliferation, apoptosis and differentiation. Of all the MLK family members, MLK3 is the best characterized in terms of its biological functions in proliferation, migration, invasion, and metastasis which makes it an attractive pharmacological target for cancer therapies (Chadee, D.N., Involvement of mixed lineage kinase 3 in cancer, Can. J. Physiol. Pharmacol. 91: 268-274 (2013)).

Inhibition of MLK-3 may be useful for the treatment of various cancers such as, for example, melanoma and gastrointestinal, pancreatic and breast cancer (Zhang-J et al., MLK3 promotes melanoma proliferation and invasion and is a target of microRNA-125b, Clin Exp Dermatol 2014, 39, 376-384; Velho-Set al., MLK3 gene mutations in mismatch repair deficient gastrointestinal tumours, Human Mol Genetics 2010, 19, 697-706; Chandana-SR et al., Inhibition of MLK3 Decreases Proliferation and Increases Antiproliferative Activity of Epidermal Growth Factor Receptor (EGFR) Inhibitor in Pancreatic Cancer Cell Lines, Cancer Growth and Metastasis 2010:3 1-9). MLK-3 regulates the proliferation of some tumor cell types, including human schwannoma and meningioma cells and breast cancer cells also over-express MLK-3. (Chen J, et al, MLK3 is critical for breast cancer cell migration and promotes a malignant phenotype in mammary epithelial cells, Oncogene. 2010; 29 (31): 4399-411).

Additionally, there are studies suggesting that loss of MLK3 in mice is protective against high fat high carbohydrate (HFHC) diet induced non-alcoholic steatohepatitis (NASH), in a weight-independent fashion, through attenuation of C-Jun N-terminal kinase (JNK) activation; therefore, MLK3 is a potential therapeutic target for the treatment of human NASH (Ibrahim S., et al, Mixed lineage kinase 3 deficient mice are protected against the high fat high carbohydrate diet-induced steatohepatitis, Liver International, 2014: 34: 427-437; Jiang J. X. et al, MLK3 as a regulator of disease progression in NASH, 2014, doi: 10.1111/liv.12556 and references therein).

Other studies have shown that blockade of the MLK3 pathway may interfere with the neurotoxic effect of beta amyloid oligomers (Xu Y, Hou XY, Liu Y, Zong YY, Different protection of K252a and N-acetyl-L-cysteine against amyloid-beta peptide-induced cortical neuron apoptosis involving inhibition of MLK3-MKK7-JNK3 signal cascades. J Neurosci Res. 2009 Mar; 87(4):918-27). Inhibition of the C-jun/JNK pathway blocks hyperphosphorylation of tau in cultured hippocampal neurons (Ma, QL, et al, Beta-amyloid oligomers induce phosphorylation of tau and inactivation of insulin receptor substrate via c-Jun N-terminal kinase signaling: suppression by omega-3 fatty acids and curcumin, J. Neurosci. 2009 Jul 15; 29 (28): 9078-89). Therefore, MLK-3 inhibitors may be of value in the treatment of Alzheimer disease and other neurodegenerative diseases involving the C-jun/JNK pathway.

On the other hand, it is known that Janus kinases (JAKs) are a family of intracellular, nonreceptor tyrosine kinases which are important signal transducers of many cytokines, growth factors and interferon. In recent years, it has been found that there is a significant enhancement in the expression of JAKs in cancer cells and cells transfected with oncogenes. It has also been described that the expression of JAKs has a close relationship with inflammation and autoimmune diseases and immune rejection of transplants. (Aggarwal, B B et al, Signal Transducer and Activator of Transcription-3, Inflammation, and Cancer How Intimate Is the Relationship? Ann. N.Y. Acad. Sci. 1171: 59-76 (2009) and references therein).

JAKs are a family of non-receptor tyrosine kinases that are relatively large molecules. There are four family members of JAKs: JAK1, JAK2, JAK3 and TYK2. JAK1, JAK2 and TYK2 exist in various tissues and cells, while JAK3 only exists in the marrow and lymphatic system. JAKs transmit extracellular stimuli through signals that are generated by the relevant receptors. Receptors and/or JAKs selectively activate signal transduction and signal transducer and activator of transcription (STAT) proteins by different phosphorylation sites. (Jiang JJJ et al, Advances in the Inhibitors of Janus Kinase, Med Chem, 2014, 4: 540-548 and references therein).

Selective inhibition of JAK kinases within the JAK family has been a desired goal of researchers in order to maximize efficacy while minimizing undesired off-target effects and to understand the role in disease of individual JAK isoforms and provide the most effective therapy for each indication. Delineation of the role of each kinase becomes possible with selective small-molecule inhibitors, but they must be selective within the kinome as well as the JAK family. This challenge is not trivial. The homology between the JAK kinases is high and the similarities in their ATP binding sites considerable. Despite these formidable obstacles, recent progress in the field has been impressive. There are now selective inhibitors for each of the JAK family members, with the expectation of seeing some of them entering the clinic in the near future (B.W. Dymock et al, Selective JAK inhibitors, Future Med Chem 2014, 6, 1439).

In the specific case of JAK3, it is a key cell signalling molecule in the immune response, which is specifically distributed in the lymphatic system; in which interleukin-2 (IL2) can activate JAK3 within a very short period of time. After a period of signal transduction, JAK3 can dephosphorylate and become inactive, so that signals generating quenching facilitate the next round of stimulus signal transmission. Thus, the inhibition of JAK3 activity will prevent side effects caused by damage to other tissues. (Jiang JJJ et al, Advances in the Inhibitors of Janus Kinase, Med Chem, 2014, 4: 540-548 and references therein).

Currently, several JAK inhibitors small molecules have been developed with promising results. One of them, Tofacitinib, is a potent inhibitor of JAK3 and JAK1 with some activity against JAK2. It has been approved in several countries for the treatment of arthritis rheumatoid (RA), and is in advanced clinical phases for the treatment of patients with moderate-to-severe psoriasis. (Ghoreschi, K et al, Jakpot! New small molecules in autoimmune and inflammatory diseases, Experimental Dermatology, 2014, 23, 7-11) y (Chiricozzi A et al, Tofacitinib for the treatment of moderate-to-severe psoriasis, Expert Rev. Clin. Immunol. Early online, 1-13 (2015)).

Others JAKs inhibitors are in clinical phases for the treatment of rheumatoid arthritis (RA), among other conditions. For example, VX-509 (decernotinib) which is in clinical phase has shown improved the signs and symptoms of RA at weeks 12 and 24 compared with the placebo group when it was administered in combination with methotrexate. Safety signals included infection and increases in liver transaminase and lipid levels (Genovese M. C et al, VX-509 (Decernotinib), an Oral Selective JAK-3 Inhibitor, in Combination with Methotrexate in Patients with Rheumatoid Arthritis, ARTHRITIS & RHEUMATOLOGY, Vol. 68, No. 1, pp 46-55 January 2016).

Accordingly, it is expected that inhibition of JAK3 could lead to the prevention and treatment of rejection and graft versus host disease (GvHD) in organ transplantation, rheumatoid arthritis, multiple sclerosis, systemic lupus erythematosus, Sjögren syndrome, Behcet's disease, type I diabetes mellitus, autoimmune thyroiditis, idiopathic thrombocytopenic purpura, ulcerative colitis, Crohn's disease, asthma, allergic rhinitis, atopic dermatitis, contact dermatitis, urticaria, eczema, psoriasis, allergic conjunctivitis, uveitis, cancer, leukemia and the like. (EP2380877 and references therein).

On the other hand, TYK2 enzyme has demonstrated an important role for signalling transduction in response to a wide variety of cytokines, including type I IFNs, IL-6, IL-10, IL-12 and IL-23. An appropriate expression of TYK2-mediated signalling might be essential for maintaining normal immune responses although in pathological conditions they promote the production of autoimmune-associated components, which are implicated in the pathogenesis of autoimmune diseases, such as rheumatoid arthritis, systemic lupus erythematosus, multiple sclerosis. Aberrant expression of TYK2 has been observed in many autoimmune conditions. (Yan Liang et al, Therapeutic potential of tyrosine kinase 2 in autoimmunity, Expert Opin. Ther. Targets (2014) 18(5):571-580). There are data supporting the idea of selective TYK2 inhibitors may be potential new therapies for the treatment of psoriasis and IBD without undesired broad immunosuppression (Dymock B W et al, Selective JAK inhibitors, Future Med. Chem. (2014) 6(12), 1439-1471).

Despite the high level of interest in selective JAK inhibitors and their therapeutic potential, TYK2 remains the least explored member of this family. Only few disclosures claiming selective TYK2 inhibitors have been published to date and no TYK2-selective inhibitors are known to be in clinical trial. The only molecule claiming TYK2 inhibition currently in a clinical trial is a compound from Pfizer: pan-inhibitor PF-06263726 (topical, psoriasis). (Menet C J, Toward selective TYK2 inhibitors as therapeutic agents for the treatment of inflammatory diseases, Pharm. Pat. Anal. (2014) 3(4), 449-466).

Taking the above into account, most of the JAK inhibitors developed so far are selective for other kinases, but, as mentioned above, do not discriminate well among the JAK family members. Such promiscuity in inhibition often leads to concerns of toxicity and with unacceptable side effects; however, it seems that the toxicity of the JAK inhibitors is limited although their long-term toxicity has not been fully determined. Therefore, the generation of highly selective inhibitors, with no off-target activity against other JAKs, may result in increased efficacy and safety. (Ghreschi K, et al, Jakpot! New small molecules in autoimmune and inflammatory diseases, Experimental Dermatology, 2014, 23, 7-11). Particularly, in the case of JAK2, due to its role in several physiological essential processes, such as erythropoiesis and neutrophil functions, to avoid its inhibition is particularly desirable. (Goedken ER et al, Tricyclic Covalent Inhibitors Selectively Target Jak3 through an Active Site Thiol, THE JOURNAL OF BIOLOGICAL CHEMISTRY VOL. 290, NO. 8, pp. 4573-4589, February 20, 2015).

The authors of the present invention have developed new carboxylic acids derivatives as potent and selective inhibitors of at least one protein kinases belonging to the family Mixed-lineage kinase (MLK), specifically of Mixed-lineage kinase 3 (MLK3) or to the family of Janus kinases especially JAK3 and TYK2.

### SUMMARY OF THE INVENTION

In one of its aspects (aspect 1), the present invention refers to novel carboxylic acid derivatives conveniently substituted of formula (I): 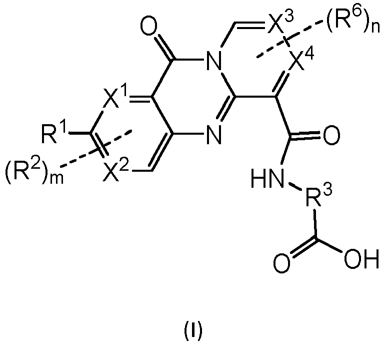 wherein:
- R¹ represents a group selected from:
   a) C₃-C₆ cycloalkyl optionally substituted by linear or branched C₁-C₆ alkyl, linear or branched C₁-C₄ alkoxy and halogen atom,
   b) C₃-C₆ heterocyclic ring containing 1 or 2 heteroatoms selected from N, O and S and which is optionally substituted by halogen atom, linear or branched C₁-C₆ haloalkyl, linear or branched C₁-C₆ alkyl, C₃-C₄ cycloalkyl, cyano group, -COOH, -CONH₂, SO₂NR₄R₅, -SO₂CH₃, NH₂, -NHC(O)R₄ and linear or branched C₁-C₄ alkoxy,
   c) halogen atom,
   d) hydrogen atom,
   e) cyano group,
   f) C₁-C₃ alkoxy, optionally substituted by 1, 2 or 3 halogen atoms,
   g) -OH,
   h) phenyl ring optionally substituted by a group selected from halogen atom, linear or branched C₁-C₆ haloalkyl, linear or branched C₁-C₆ alkyl, C₃-C₄ cycloalkyl, cyano group, - COOH, -CONH₂, SO₂NR₄R₅, -SO₂CH₃, NH₂, -NHC(O)R₄ and linear or branched C₁-C₄ alkoxy,
   i) -S(O)ₚR⁷, wherein R⁷ is C₁-C₃ alkyl and p is an integer selected from 1 to 2, and
   j) linear or branched C₁-C₆ alkyl optionally substituted by 1, 2 or 3 halogen atoms,
- each one of R² and R⁶ independently represents a group selected from:
   a) halogen atom,
   b) linear or branched C₁-C₆ alkyl,
   c) linear or branched C₁-C₆ haloalkyl, and
   d) phenyl or C₄-C₆ heterocyclic ring containing 1 or 2 heteroatoms selected from N, O and S and which are optionally substituted by a group selected from halogen atom, linear or branched C₁-C₆ haloalkyl, linear or branched C₁-C₆ alkyl and linear or branched C₁-C₄ alkoxy,
   e) C₃-C₆ cycloalkyl optionally substituted by linear or branched C₁-C₆ alkyl and linear or branched C₁-C₄ alkoxy
      wherein the group R², if present, replaces the hydrogen atom of one of the groups CHin the ring which contains X¹ and X² and R⁶, if present, replaces the hydrogen atom of one of the groups CH- in the ring which contains X³ and X⁴,
- m and n are integers independently selected from 0 and 1,
- R³ represents a group selected from:
   a) 4- to 10- membered, saturated cycle optionally containing 1 or 2 heteroatoms selected from N and O, which is optionally substituted by 1, 2 or 3 groups selected from linear or branched C₁-C₆ alkyl, linear or branched C₁-C₆ alkoxy, -OH and - NR⁴R⁵,
   b) linear or branched C₁-C₆ alkyl,
- R⁴ and R⁵ represent independently a group selected from hydrogen atom, linear or branched C₁-C₆ alkyl and C₃-C₆ cycloalkyl group,
- X¹ and X² are independently selected from CH and N with the condition that either none or one of them is N,
- X³ and X⁴ are independently selected from CH and N with the condition that either none or one of them is N,
and pharmaceutically acceptable salts thereof.

In a second aspect the present invention relates to processes for the preparation of the compounds of aspect 1.

In a third aspect the present invention relates to pharmaceutical compositions comprising a compound of aspect 1 and a pharmaceutical aspect diluent or carrier.

In a fourth aspect the present invention relates to pharmaceutical compositions according to the third aspect described above which further comprise a therapeutically effective amount of a therapeutic agent selected from agent useful for the treatment of liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among others.

In a fifth aspect the present invention relates to the use of the compound of aspect 1 in the manufacture of a medicament for the treatment of a disease or pathological condition that can be ameliorated by inhibition of at least one enzyme selected from the group consisting of MLK kinases, particularly MLK3 and Janus kinases selected from JAK3 and TYK2, such as liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among other.

In a sixth aspect the present invention relates to methods for the treatment of diseases that can be ameliorated by inhibition of at least one enzyme selected from the group consisting of MLK kinases, particularly MLK3 and Janus kinases selected from JAK3 and TYK2 by administration of the compounds of the first aspect or pharmaceutical compositions of the second and third aspects described above to a subject in need of said treatment; the diseases are selected from liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among others.

In a seventh aspect the present invention relates to a combination product of the compound of the first aspect described above with one more therapeutic agent known to be useful in the treatment of diseases selected from such as liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among others.

In an eighth aspect the present invention relates to the compound of aspect 1 for use in the treatment of a disease or pathological condition that can be ameliorated by inhibition of at least one enzyme selected from the group consisting of MLK kinases, particularly MLK3 and Janus kinases selected from JAK3 and TYK2, such as liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among other.

As it is said before, the carboxylic acids derivatives of the present invention are useful in the treatment or prevention of diseases known to be susceptible to amelioration by treatment with inhibitor of at least one enzyme selected from the group consisting of MLK kinases, particularly MLK3 and Janus kinases selected from JAK3 and TYK2 such as liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric cancer, lung cancer, pancreatic cancer, breast cancer, colon cancer, colorectal cancer, and others diseases selected from asthma, chronic obstructive pulmonary disease (COPD), transplant rejection, haematological disease, uveitis, dry eye and allergic conjunctivitis and neurodegenerative diseases including Alzheimer disease, among others. In a preferred embodiment, the compounds of formula (I) due to their moderate to high systemic exposure after oral administration, are especially suited for the treatment of diseases selected from non-alcoholic steatohepatitis (NASH), rheumatoid arthritis, multiple sclerosis, psoriasis, cancer including gastric cancer, lung cancer, pancreatic cancer, breast cancer, colon cancer, colorectal cancer, transplant rejection, haematological diseases.

Accordingly, the derivatives of the present invention and pharmaceutically acceptable salts thereof, and pharmaceutical compositions comprising such compounds and / or salts thereof, may be used in a method of treatment of pathological conditions or disease of human body which comprises administering to a subject in need of said treatment, an effective amount of the carboxylic acid derivative of the invention or a pharmaceutically acceptable salt thereof.

As used herein, the term Cₐ-C_{b} cycloalkyl embraces hydrocarbon cyclic groups having a to b carbon atoms. Such cycloalkyl groups include, for example, cyclopropyl, cyclobutyl, cyclopentyl and cyclohexyl.

As used herein, the term Cₐ-C_{b} alkyl includes linear or branched radicals, having from 1 to 6 carbon atoms. Preferred radicals include 1 to 4 carbon atoms. Examples of linear or branched alky groups are methyl, ethyl, n-propyl, iso-propyl, n-butyl, i-butyl, sec-butyl, tert-buty, pentyl and hexyl.

As used herein, the term linear or branched Cₐ-C_{b} alkoxy is used to designate radicals which contain linear or branched Cₐ-C_{b} alkyl radicals linked to an oxygen atom (CₓH₂ₓ₊₁-O-). Preferred alkoxy radicals include for example, methoxy, ethoxy, n-propoxy, i-propoxy.

As used herein, the term a 4- to 10- membered, saturated cycle embraces ring systems of 4 to 10 members containing carbon atoms and optionally 1 or 2 heteroatoms selected from N and O. Said ring systems may be monocyclic or polycyclic and the polycyclic ring system include systems with fused rings (i.e. rings sharing two ring atoms), bridged rings (i.e. rings sharing more than two ring atoms) and spiranic systems (i.e. wherein two rings share only one ring atom) Said cycles include, by way of example, the following monocyclic ring systems: cyclopentyl, cyclohexyl, tetrahydropyranyl, tetrahydrofuranyl, and piperidinyl, and the following polycyclic bridged ring systems: bicyclo[2.2.1]heptanyl, 7-aza-bicyclo[2.2.1]heptanyl, (bicyclo[2.2.2]octanyl) and adamantyl. Said cycles are optionally substituted by 1, 2 or 3 substituents selected from linear or branched C₁-C₆ alkyl, linear or branched C₁-C₆ alkoxy, halogen atom, linear or branched C₁-C₆ haloalkyl, -OH and amines. The substituents of the 4- to 10- membered cycle may be replacing a hydrogen atom of any of the carbon atoms in the cycle or a hydrogen atom of any of the nitrogen atoms in the cycle. In an embodiment, the cycle is a 5- to 8-membered carbocycle.

In another embodiment, the 4- to 10- membered, saturated, polycyclic ring systems comprise two or more fused or bridged rings each consisting of 3 to 7 atoms, wherein 1 or 2 atoms can be heteroatoms selected from N and O.

When, in the compounds of formula (I), R³ represents a polycyclic ring system the carbon atom of the carboxylic acid group (-COOH) and the nitrogen atom of the amido group (-CONH-) may be linked to the same ring or to different rings of the polycyclic ring system.

As used herein, the term Cₐ-C_{b} heterocyclic rings embrace ring system of a to b carbon atoms containing 1 or 2 heteroatoms selected from N, O and S forming part of the ring. This definition refers to a particular subgroup of 3- to 10- membered, cycles mentioned above. Such rings include, for example, pyridinyl, tetrahydropyranyl, pyperazinyl, morpholinyl. Said rings are optionally substituted by 1, 2 or 3 substituents selected from halogen atom, linear or branched C₁-C₆ haloalkyl, linear or branched C₁-C₆ alkyl, C₃-C₄ cycloalkyl, cyano group, -COOH,-CONH₂, SO₂NR₄R₅, -SO₂CH₃, NH₂, -NHC(O)R₄ and linear or branched C₁-C₄ alkoxy. The substituents of the heterocyclic ring may be replacing a hydrogen atom of any of the carbon atoms in the ring or a hydrogen atom of any of the nitrogen atoms in the ring.

In a particular embodiment the Cₐ-C_{b} heterocyclic ring is a saturated ring system of a to b carbon atoms containing 1 or 2 heteroatoms selected from N and O forming part of the ring and more particularly 4- to 10- membered, saturated cycles such as tetrahydropyranyl, pyperazinyl and morpholinyl. Said rings are optionally substituted by 1, 2 or 3 substituents selected from halogen atom, linear or branched C₁-C₆ haloalkyl, linear or branched C₁-C₆ alkyl and linear or branched C₁-C₄ alkoxy wherein said substituents of the heterocyclic ring may be replacing a hydrogen atom of any of the carbon atoms in the ring or a hydrogen atom of any of the nitrogen atoms in the ring.

The core ring system of the compounds of the invention may be depicted as shown below:

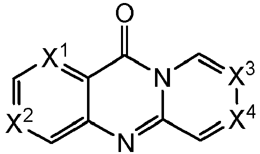

Said core ring system may be selected among the following ring systems:

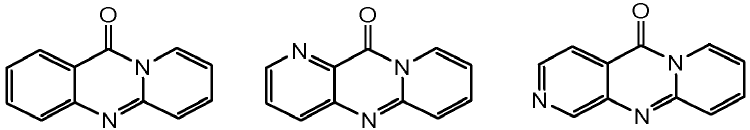

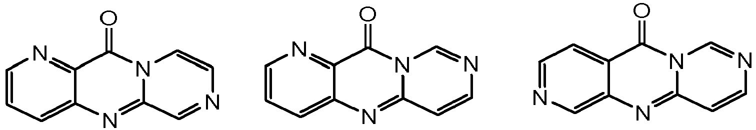

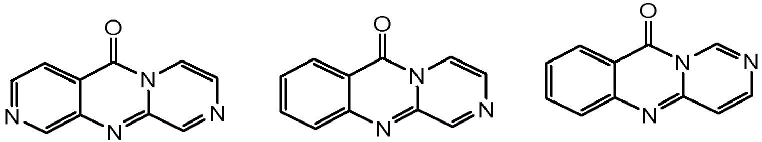

As used herein, the term halogen atom includes chlorine, fluorine, bromine and iodine atoms, preferably fluorine, chlorine and bromine atoms. The term halo, when used as a prefix, has the same meaning. As a mere example haloalkyl means an alkyl substituted by one or more halogen atoms.

As used herein, some of the atoms, radicals, chains or cycles present in the general structures of the invention are "optionally substituted". This means that these atoms, radicals, chains or cycles can be either unsubstituted or substituted in any position by one or more, for example 1, 2, 3 or 4, substituents, whereby the hydrogen atoms bound to the unsubstituted atoms, radicals, chains or cycles are replaced by chemically acceptable atoms, radicals, chains or cycles. When two or more substituents are present, each substituent may be the same or different.

As used herein, the term pharmaceutically acceptable salt is used to designate salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium), alkali earth metal (e.g. calcium or magnesium) hydroxides, and organic bases, for example alkyl amines, arylalkyl amines and heterocyclic amines.

Other preferred salts according to the invention are quaternary ammonium compounds wherein an equivalent of an anion (X⁻ⁿ) is associated with the positive charge of the N atom. X⁻ⁿ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulfonate and p-toluenesulphonate. X⁻ⁿ is preferably an anion selected from chloride, bromide, iodide, sulphate, nitrate, acetate, maleate, oxalate, succinate or trifluoroacetate. More preferably, X- is chloride, bromide, trifluoroacetate or methanesulfonate.

In a particular embodiment the first to eight aspects of the present invention refers to compounds of formula (I) wherein:
- R¹ represents a group selected from:
   a) C₃-C₆ cycloalkyl optionally substituted by linear or branched C₁-C₆ alkyl and linear or branched C₁-C₄ alkoxy,
   b) C₄-C₆ heterocyclic ring containing 1 or 2 heteroatoms selected from N, O and S and which is optionally substituted by halogen atom, linear or branched C₁-C₆ haloalkyl, linear or branched C₁-C₆ alkyl and linear or branched C₁-C₄ alkoxy,
   c) halogen atom,
   d) hydrogen atom,
   e) cyano group,
   f) C₁-C₃ alkoxy, optionally substituted by 1, 2 or 3 halogen atoms,
   g) -OH,
   h) phenyl ring optionally substituted by a group selected from halogen atom, linear or branched C₁-C₆ haloalkyl, linear or branched C₁-C₆ alkyl and linear or branched C₁-C₄ alkoxy,
   i) -S(O)ₚR⁷, wherein R⁷ is C₁-C₃ alkyl and p is an integer selected from 1 to 2, and
   j) linear or branched C₁-C₆ alkyl optionally substituted by 1, 2 or 3 halogen atoms,
- each one of R² and R⁶ independently represents a group selected from:
   f) halogen atom,
   g) linear or branched C₁-C₆ alkyl,
   h) linear or branched C₁-C₆ haloalkyl, and
   i) phenyl or C₄-C₆ heterocyclic ring containing 1 or 2 heteroatoms selected from N, O and S and which are optionally substituted by a group selected from halogen atom, linear or branched C₁-C₆ haloalkyl, linear or branched C₁-C₆ alkyl and linear or branched C₁-C₄ alkoxy,
   j) C₃-C₆ cycloalkyl optionally substituted by linear or branched C₁-C₆ alkyl and linear or branched C₁-C₄ alkoxy
      wherein the group R², if present, replaces the hydrogen atom of one of the groups CHin the ring which contains X¹ and X² and R⁶, if present, replaces the hydrogen atom of one of the groups CH- in the ring which contains X³ and X⁴,
- m and n are integers independently selected from 0 and 1,
- R³ represents a group selected from:
   c) 4- to 10- membered, saturated cycle optionally containing 1 or 2 heteroatoms selected from N and O, which is optionally substituted by 1, 2 or 3 groups selected from linear or branched C₁-C₆ alkyl, linear or branched C₁-C₆ alkoxy, -OH and - NR⁴R⁵,
   d) linear or branched C₁-C₆ alkyl,
- R⁴ and R⁵ represent independently a group selected from hydrogen atom, linear or branched C₁-C₆ alkyl and C₃-C₆ cycloalkyl group,
- X¹ and X² are independently selected from CH and N with the condition that either none or one of them is N,
- X³ and X⁴ are independently selected from CH and N with the condition that either none or one of them is N,
and pharmaceutically acceptable salts thereof.

According to one embodiment of the present invention in the compounds of formula (I), R¹ represents a group selected from:
- C₃-C₆ cycloalkyl optionally substituted by linear or branched C₁-C₆ alkyl and linear or branched C₁-C₄ alkoxy,
- C₄-C₆ heterocyclic ring containing 1 or 2 heteroatoms selected from N and O and which is optionally substituted by linear or branched C₁-C₆ alkyl,
- phenyl ring optionally substituted by a group selected from halogen atom, linear or branched C₁-C₆ haloalkyl, linear or branched C₁-C₆ alkyl and linear or branched C₁-C₄ alkoxy.

In a more preferred embodiment, R¹ represents a cyclopropyl group optionally substituted by a group selected from linear or branched C₁-C₆ alkyl and linear or branched C₁-C₄ alkoxy.

In a more preferred embodiment, R¹ represents a group selected from pyridinyl, piperazinyl and morpholinyl group optionally substituted by a linear or branched C₁-C₆ alkyl group.

In another preferred embodiment, R¹ represents a phenyl ring optionally substituted by a group selected from halogen atom and linear or branched C₁-C₆ haloalkyl.

According to another embodiment of the present invention in the compound of formula (I) m and n have a value of 0.

According to another embodiment of the present invention in the compounds of formula (I), R³ represents a C₅-C₆ cycloalkyl optionally substituted by a group selected from linear or branched C₁-C₆ alkyl and -OH.

In a preferred embodiment, R³ represents a group selected from cyclopentyl and cyclohexyl group optionally substituted by a group selected from linear or branched C₁-C₆ alkyl and -OH. In a more preferred embodiment, R³ represents a cyclohexyl group substituted by a group selected from linear or branched C₁-C₆ alkyl and -OH.

According to another embodiment of the present invention in the compounds of formula (I), X¹, X², X³ and X⁴ represent CH.

According to another embodiment of the present invention in the compounds of formula (I), R⁴ and R⁵ represent a hydrogen atom.

According to one embodiment of the present invention in the compounds of formula (I), R¹ represents a cyclopropyl group optionally substituted by a group selected from linear or branched C₁-C₆ alkyl and linear or branched C₁-C₄ alkoxy, each one of m and n have a value of 0, R³ represents a cyclohexyl group optionally substituted by a group selected from linear or branched C₁-C₆ alkyl and -OH and X¹, X², X³ and X⁴ represent CH.

Particular individual compounds of the present invention include:
4-(2-cyclopropyl-11-oxo-11H-pyrido[2,1-b]quinazoline-6-carboxamido)cyclohexane-1-carboxylic acid
(1s,4s)-4-(2-(5-methylthiophen-2-yl)-11-oxo-11H-pyrido[2,1-b]quinazoline-6-carboxamido)cyclohexane-1-carboxylic acid
(1s,4s)-4-(2-cyclopropyl-11-oxo-11H-pyrido[2,1-b]quinazoline-6-carboxamido)cyclohexane-1-carboxylic acid
(1r,4r)-4-(2-cyclopropyl-11-oxo-11H-pyrido[2,1-b]quinazoline-6-carboxamido)cyclohexane-1-carboxylic acid
(1s,4s)-4-(11-oxo-2-(trifluoromethoxy)-11H-pyrido[2,1-b]quinazoline-6-carboxamido)cyclohexane-1-carboxylic acid
4-(2-cyclopropyl-11-oxo-11H-pyrido[2,1-b]quinazoline-6-carboxamido)bicyclo[2.2.2]octane-1-carboxylic acid
4-(2-cyclopropyl-11-oxo-11H-pyrido[2,1-b]quinazoline-6-carboxamido)bicyclo[2.2.1]heptane-1-carboxylic acid
1-(2-cyclopropyl-11-oxo-11H-pyrido[2,1-b]quinazoline-6-carboxamido)cyclopentane-1-carboxylic acid
2-(2-cyclopropyl-11-oxo-11H-pyrido[2,1-b]quinazoline-6-carboxamido)-2-methylpropanoic acid
4-(8-methyl-11-oxo-11H-pyrido[2,1-b]quinazoline-6-carboxamido)cyclohexane-1-carboxylic acid
(1s,4s)-4-(8-methyl-11-oxo-11H-pyrido[2,1-b]quinazoline-6-carboxamido)cyclohexane-1-carboxylic acid
4-(11-oxo-11H-pyrido[2,1-b]quinazoline-6-carboxamido)cyclohexane-1-carboxylic acid
(1s,4s)-4-(11-oxo-11H-pyrido[2,1-b]quinazoline-6-carboxamido)cyclohexane-1-carboxylic acid
(1s,4s)-4-(2-morpholino-11-oxo-11H-pyrido[2,1-b]quinazoline-6-carboxamido)cyclohexane-1-carboxylic acid
(1s,4s)-4-(11-oxo-2-phenyl-11H-pyrido[2,1-b]quinazoline-6-carboxamido)cyclohexane-1-carboxylic acid
(1s,4s)-4-(2-(4-fluorophenyl)-11-oxo-11H-pyrido[2,1-b]quinazoline-6-carboxamido)cyclohexane-1-carboxylic acid
(1s,4s)-4-(11-oxo-2-(pyridin-4-yl)-11H-pyrido[2,1-b]quinazoline-6-carboxamido)cyclohexane-1-carboxylic acid
(1s,4s)-4-(2-cyclopropyl-8-methyl-11-oxo-11H-pyrido[2,1-b]quinazoline-6-carboxamido)cyclohexane-1-carboxylic acid
4-(2-hydroxy-11-oxo-11H-pyrido[2,1-b]quinazoline-6-carboxamido)cyclohexane-1-carboxylic acid
4-(2-methoxy-11-oxo-11H-pyrido[2,1-b]quinazoline-6-carboxamido)cyclohexane-1-carboxylic acid
4-(2-cyano-11-oxo-11H-pyrido[2,1-b]quinazoline-6-carboxamido)cyclohexane-1-carboxylic acid
4-(2-fluoro-11-oxo-11H-pyrido[2,1-b]quinazoline-6-carboxamido)cyclohexane-1-carboxylic acid
4-(2-chloro-11-oxo-11H-pyrido[2,1-b]quinazoline-6-carboxamido)cyclohexane-1-carboxylic acid
4-(2-bromo-11-oxo-11H-pyrido[2,1-b]quinazoline-6-carboxamido)cyclohexane-1-carboxylic acid
3-(11-oxo-11H-pyrido[2,1-b]quinazoline-6-carboxamido)cyclohexane-1-carboxylic acid
(1r,4r)-4-(8-methyl-11-oxo-11H-pyrido[2,1-b]quinazoline-6-carboxamido)cyclohexane-1-carboxylic acid
(1s,4s)-4-(2-cyclopentyl-11-oxo-11H-pyrido[2,1-b]quinazoline-6-carboxamido)cyclohexane-1-carboxylic acid
(trans)-4-(2-(4-fluorophenyl)-11-oxo-11H-pyrido[2,1-b]quinazoline-6-carboxamido)cyclohexane-1-carboxylic acid
(cis)-4-(2-(3-fluorophenyl)-11-oxo-11H-pyrido[2,1-b]quinazoline-6-carboxamido)cyclohexane-1-carboxylic acid
(cis)-4-(2-(3,4-difluorophenyl)-11-oxo-11H-pyrido[2,1-b]quinazoline-6-carboxamido)cyclohexane-1-carboxylic acid
(cis)-4-(2-(4-cyanophenyl)-11-oxo-11H-pyrido[2,1-b]quinazoline-6-carboxamido)cyclohexane-1-carboxylic acid
(cis)-4-(2-(3-cyanophenyl)-11-oxo-11H-pyrido[2,1-b]quinazoline-6-carboxamido)cyclohexane-1-carboxylic acid
4-(6-(((cis)-4-carboxycyclohexyl)carbamoyl)-11-oxo-11H-pyrido[2,1-b]quinazolin-2-yl)benzoic acid
(cis)-4-(11-oxo-2-(pyridin-3-yl)-11H-pyrido[2,1-b]quinazoline-6-carboxamido)cyclohexane-1-carboxylic acid
(trans)-4-(11-oxo-2-(pyridin-4-yl)-11H-pyrido[2,1-b]quinazoline-6-carboxamido)cyclohexane-1-carboxylic acid
(cis)-4-(2-(3-fluoropyridin-4-yl)-11-oxo-11H-pyrido[2,1-b]quinazoline-6-carboxamido)cyclohexane-1-carboxylic acid
(cis)-4-(11-oxo-2-(pyrimidin-5-yl)-11H-pyrido[2,1-b]quinazoline-6-carboxamido)cyclohexane-1-carboxylic acid
(cis)-4-(2-(1-methyl-1H-pyrazol-4-yl)-11-oxo-11H-pyrido[2,1-b]quinazoline-6-carboxamido)cyclohexane-1-carboxylic acid
(cis)-4-(11-oxo-2-(thiophen-2-yl)-11H-pyrido[2,1-b]quinazoline-6-carboxamido)cyclohexane-1-carboxylic acid
(cis)-4-(8-bromo-11-oxo-11H-pyrido[2,1-b]quinazoline-6-carboxamido)cyclohexane-1-carboxylic acid
3-(2-cyclopropyl-11-oxo-11H-pyrido[2,1-b]quinazoline-6-carboxamido)cyclobutane-1-carboxylic acid
(1s,4s)-4-(2-(5-cyanothiophen-2-yl)-11-oxo-11H-pyrido[2,1-b]quinazoline-6-carboxamido)cyclohexane-1-carboxylic acid
(1s,4s)-4-(2-(5-carbamoylthiophen-2-yl)-11-oxo-11H-pyrido[2,1-b]quinazoline-6-carboxamido)cyclohexane-1-carboxylic acid
(1s,4s)-4-(2-(1-methyl-1H-imidazol-4-yl)-11-oxo-11H-pyrido[2,1-b]quinazoline-6-carboxamido)cyclohexane-1-carboxylic acid
(1s,4s)-4-(2-(5-cyclopropylthiophen-2-yl)-11-oxo-11H-pyrido[2,1-b]quinazoline-6-carboxamido)cyclohexane-1-carboxylic acid
(1s,4s)-4-(11-oxo-2-(thiazol-5-yl)-11H-pyrido[2,1-b]quinazoline-6-carboxamido)cyclohexane-1-carboxylic acid
5-(6-(((1s,4s)-4-carboxycyclohexyl)carbamoyl)-11-oxo-11H-pyrido[2,1-b]quinazolin-2-yl)thiophene-2-carboxylic acid
and pharmaceutically acceptable salts thereof.

The compounds of the present invention can be prepared by using the procedures described below. To facilitate the description of the procedures, concrete examples have been used but they do not restrict in any way the scope of the present invention. The synthesis of compound of formula (I) is outlined in scheme 1.

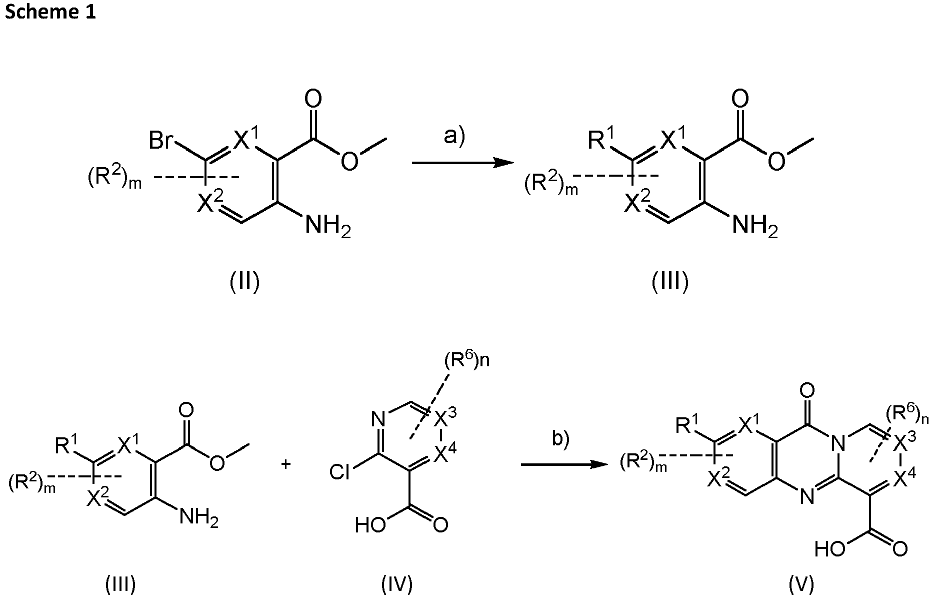

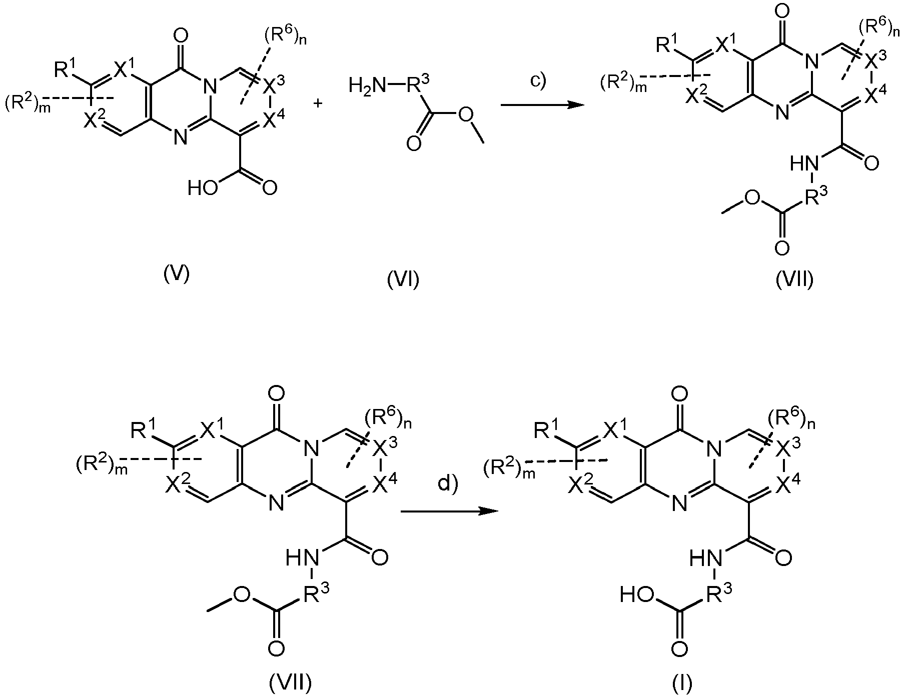

In the above scheme, compounds of formula (I) are compounds according to the present invention wherein R¹, R², R³, R⁶, X¹, X², X³ and X⁴ are as hereinabove defined.

Reagents and conditions:
Step a) R¹-B(OH)₂, Pd-Cat., or Secondary cyclic or heterocyclic amine
Step b) HCl, Isopropanol 24h, 100ºC.
Step c) EDC, HOBt, EDIA, DMF, room temperature.
Step d) NaOH, H₂O/THF or HCl, AcOH/H₂O.

Derivatives of general formula (V) are prepared from commercially available optionally substituted 2-aminobenzic acids or ethyl or methyl optionally substituted 2-aminobenzoates or the correspondent optionally substituted amino-heteroaryl carboxylates (III) and optionally substituted 2-chloronicotinic acids or optionally substituted chlorheteroarylic acids (IV), according to Scheme 1. In some cases, that reagents (III) are not available, they can be obtained, for example, through the substitution of the bromide atom from the corresponding carboxylic acids or carboxylates of formula (II). The starting reagents (III) and (IV) are reacted by a cyclization reaction in acidic condition in isopropanol or xylene at temperatures between 80º and 110ºC to provide acids of formula (V). The reaction of these acids with amines (VI) in polar aprotic solvents such as DCM, THF, Acetonitrile or DMF in the presence of coupling reagent such as HATU, EDC, HOBt or T3P and temperatures ranging from 0ºC to 80ºC affords the ester derivatives of formula (VII) and their successive hydrolyse under both basic and acid condition provides compounds of formula (I), which are the object of the present invention.

Another way to obtain in certain positions substituted compounds of formula (I) is achieved, for example, through the nucleophilic substitution of brominated derivatives (VIII) and (X) with amines or through the coupling reaction of these derivatives, for instance with aryl or heteroaryl boronic acids under Suzuki conditions according to scheme 2. Such compounds of formula (Ia) and (Ib) are particular cases of the present invention.

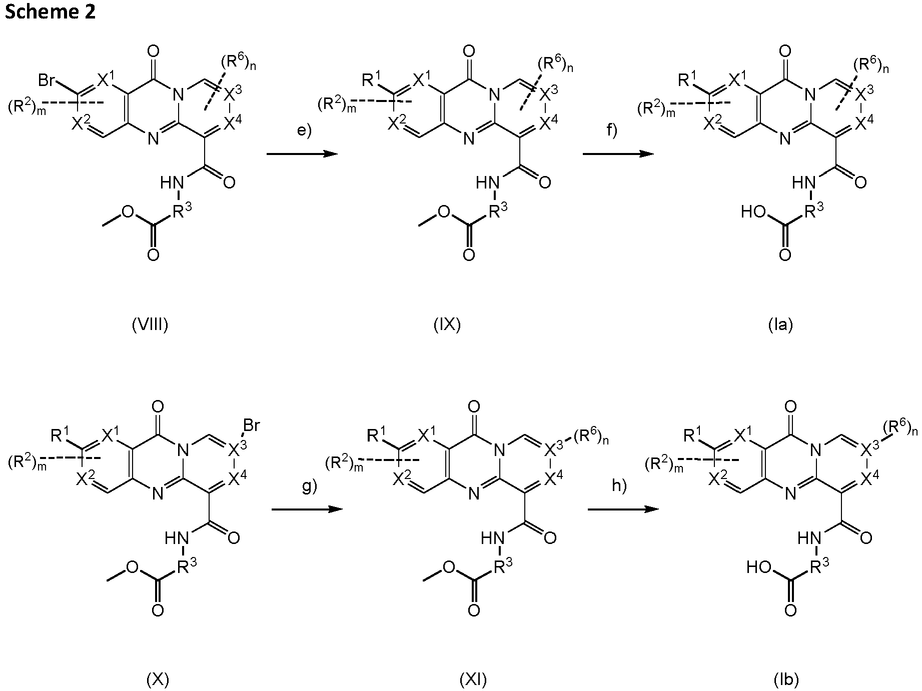

Reagents and conditions:
Step e) and g) R¹-B(OH)₂, Pd-Cat.; or Secondary cyclic or heterocyclic amines
Step f) and h) NaOH, H₂O/THF; or HCl, AcOH/H₂O.

In the above scheme, compounds of formula (I) are compounds according to the present invention wherein R¹, R², R³, R⁶, X¹, X², X³ and X⁴ are as hereinabove defined.

### Pharmacological activity

### Functional assays of Protein kinases

The functional assays of protein kinases were carried out in 384-well plates using a final volume of 30 µl. The reaction begins through the combination between the kinase enzyme and the peptic substrate (indicated in the table wherein the prefix 5-FAM indicates that the amino terminal group of the peptide is linked to 5-carboxyfluorescein and CONH₂ indicates that the carboxylic acid terminal group is amidated) in a concentration of 1.5 µM in presence of ATP and non-ATP controls. The reaction was reads in a "Caliper EzReader LabChip 3000" (Caliper, Hopkinton, MA) reader, based on electrophoretic mobility of the fluorescent substrate and the phosphorylated product.

The inhibition percentages were calculated by comparison between control reactions, for 100% of inhibition and reactions with only DMSO for 0% of inhibition. The reaction conditions were the following:

| **Enzymes** | **Substrate** | **Buffer** | **ATP Concentration** | **Incubation (min)** |
|---|---|---|---|---|
| JAK1 (Product No.: 08-144, Carna Biosciences) | 5-FAM-KKSRGDYMTMQIG-CONH₂ (5-FAM-SEQ ID NO: 1-CONH₂) | 100mM Hepes pH=7.2, 0.015% Brij-35, 4mM DTT, 2%DMSO, 10mM MgCl₂. | 100 µM | 150 |
| JAK2 (Product No.: 08-045, Carna Biosciences) | 5-FAM-EEPLYWSFPAKKK-CONH₂ (5-FAM-SEQ ID NO: 2-CONH₂) | 100mM Hepes pH=7.5, 4% DMSO, 0.003% Brij, 0.004% Tween 20, 10mM MgCl₂. | 300 µM | 30 |
| JAK3 (Product No.: 08-046, Carna Biosciences) | 5-FAM-EEPLYWSFPAKKK-CONH₂ (5-FAM-SEQ ID NO: 2-CONH₂) | 20m Hepes pH7.4, 0.01% BSA X-100, 0.005% Tween 20, 2% DMSO, 10mM MgCl₂. | 8 µM | 30 |
| TYK2 (Product No.: 08-147, Carna Biosciences) | 5-FAM-KKSRGDYMTMQIG-CONH₂ (5-FAM-SEQ ID NO: 1-CONH₂) | 100mM Hepes pH=7.2, 0.015% Brij-35, 4mM DTT, 2%DMSO, 10mM MgCl₂. | 20 µM | 45 |

### MLK3 activity inhibition test

Activity test was carried out using the kit from Reaction Biology (CAT#: MLK3). The test uses enzyme Human MLK3 and substrates are MBP (Myelin Basic Protein, HGNC ID:6925) 10 µM and ATP 10 µM. Other reagents are the following: Base Reaction buffer; 20 mM Hepes (pH 7.5), 10 mM MgCl₂, 1 mM EGTA, 0.02% Brij35, 0.02 mg/ml BSA, 0.1 mM Na₃VO₄, 2 mM DTT, 1% DMSO.

The reaction was carried out following the instructions of manufacturer. Briefly, kinase/substrate pairs were prepared in reaction buffer. Compounds were delivered into the reaction, followed 20 minutes later by addition of a mixture of ATP (Sigma, St. Louis MO) and ³³P ATP (Perkin Elmer, Waltham MA) to a final concentration of 10 µM. Reactions were carried out at room temperature for 120 min, followed by spotting of the reactions onto P81 ion exchange filter paper (Whatman Inc., Piscataway, NJ). Unbound phosphate was removed by extensive washing of filters in 0.75% phosphoric acid. After subtraction of background derived from control reactions containing inactive enzyme, kinase activity data was expressed as the percent remaining kinase activity in test samples compared to vehicle (dimethyl sulfoxide) reactions. IC₅₀ values and curve fits were obtained using Prism (GraphPad Software).

### Results

Table 1 shows the results of assays described below of some compounds of the present invention.

**Table 1**

| **Ex.** | **Compound** | **MLK3** | **JAK1** | **JAK2** | **JAK3** | **TYK2** |
|---|---|---|---|---|---|---|
| 1 | 4-(2-cyclopropyl-11-oxo-11H-pyrido[2,1-b]quinazoline-6-carboxamido)cyclohexane-1-carboxylic acid | - | C | C | A | B |
| 2 | (1S,4S)-4-(2-(5-methylthiophen-2-yl)-11-oxo-11H-pyrido[2,1-b]quinazoline-6-carboxamido)cyclohexane-1-carboxylic acid | - | - | - | A | A |
| 3 | (1S,4S)-4-(2-cyclopropyl-11-oxo-11H-pyrido[2,1-b]quinazoline-6-carboxamido)cyclohexane-1-carboxylic acid | A | C | C | A | A |
| 4 | (1R,4R)-4-(2-cyclopropyl-11-oxo-11H-pyrido[2,1-b]quinazoline-6-carboxamido)cyclohexane-1-carboxylic acid | - | C | C | B | C |
| 6 | 4-(2-cyclopropyl-11-oxo-11H-pyrido[2,1-b]quinazoline-6-carboxamido)bicyclo[2.2.2]octane-1-carboxylic acid | A | C | C | B | B |
| 7 | 4-(2-cyclopropyl-11-oxo-11H-pyrido[2,1-b]quinazoline-6-carboxamido)bicyclo[2.2.1]heptane-1-carboxylic acid | - | C | C | B | B |
| 8 | 4-(2-methoxy-11-oxo-11H-pyrido[2,1-b]quinazoline-6-carboxamido)cyclohexane-1-carboxylic acid | A | - | - | A | B |
| 10 | 4-(8-methyl-11-oxo-11H-pyrido[2,1-b]quinazoline-6-carboxamido)cyclohexane-1-carboxylic acid | C | C | C | B | A |
| 11 | (1S,4S)-4-(8-methyl-11-oxo-11H-pyrido[2,1-b]quinazoline-6-carboxamido)cyclohexane-1-carboxylic acid | - | C | C | C | B |
| 12 | 4-(11-oxo-11H-pyrido[2,1-b]quinazoline-6-carboxamido)cyclohexane-1-carboxylic acid | - | C | C | B | B |
| 13 | (1S,4S)-4-(11-oxo-11H-pyrido[2,1-b]quinazoline-6-carboxamido)cyclohexane-1-carboxylic acid | B | C | C | B | B |
| 14 | (1S,4S)-4-(2-morpholino-11-oxo-11H-pyrido[2,1-b]quinazoline-6-carboxamido)cyclohexane-1-carboxylic acid | A | C | C | A | A |
| 15 | (1S,4S)-4-(11-oxo-2-phenyl-11H-pyrido[2,1-b]quinazoline-6-carboxamido)cyclohexane-1-carboxylic acid | - | C | C | A | A |
| 16 | (1S,4S)-4-(2-(4-fluorophenyl)-11-oxo-11H-pyrido[2,1-b]quinazoline-6-carboxamido)cyclohexane-1-carboxylic acid | A | C | C | A | A |
| 17 | (1S,4S)-4-(11-oxo-2-(pyridin-4-yl)-11H-pyrido[2,1-b]quinazoline-6-carboxamido)cyclohexane-1-carboxylic acid | A | C | C | A | A |
| 19 | 4-(2-hydroxy-11-oxo-11H-pyrido[2,1-b]quinazoline-6-carboxamido)cyclohexane-1-carboxylic acid | - | - | - | B | B |
| 20 | 4-(2-methoxy-11-oxo-11H-pyrido[2,1-b]quinazoline-6-carboxamido)cyclohexane-1-carboxylic acid | A | - | - | A | B |
| 21 | 4-(2-cyano-11-oxo-11H-pyrido[2,1-b]quinazoline-6-carboxamido)cyclohexane-1-carboxylic acid | C | C | C | B | C |
| 23 | 4-(2-chloro-11-oxo-11H-pyrido[2,1-b]quinazoline-6-carboxamido)cyclohexane-1-carboxylic acid | - | C | C | B | B |
| 24 | 4-(2-bromo-11-oxo-11H-pyrido[2,1-b]quinazoline-6-carboxamido)cyclohexane-1-carboxylic acid | - | C | C | B | B |
| 29 | (cis)-4-(2-(3-fluorophenyl)-11-oxo-11H-pyrido[2,1-b]quinazoline-6-carboxamido)cyclohexane-1-carboxylic acid | - | - | - | A | A |
| 30 | (cis)-4-(2-(3,4-difluorophenyl)-11-oxo-11H-pyrido[2,1-b]quinazoline-6-carboxamido)cyclohexane-1-carboxylic acid | - | - | - | A | A |
| 31 | (cis)-4-(2-(4-cyanophenyl)-11-oxo-11H-pyrido[2,1-b]quinazoline-6-carboxamido)cyclohexane-1-carboxylic acid | - | - | - | A | A |
| 32 | (cis)-4-(2-(3-cyanophenyl)-11-oxo-11H-pyrido[2,1-b]quinazoline-6-carboxamido)cyclohexane-1-carboxylic acid | - | - | - | A | A |
| 33 | 4-(6-(((cis)-4-carboxycyclohexyl)carbamoyl)-11-oxo-11H-pyrido[2,1-b]quinazolin-2-yl)benzoic acid | - | - | - | A | A |
| 34 | (cis)-4-(11-oxo-2-(pyridin-3-yl)-11H-pyrido[2,1-b]quinazoline-6-carboxamido)cyclohexane-1-carboxylic acid | - | - | - | A | A |
| 35 | (trans)-4-(11-oxo-2-(pyridin-4-yl)-11H-pyrido[2,1-b]quinazoline-6-carboxamido)cyclohexane-1-carboxylic acid | - | - | - | B | C |
| 36 | (cis)-4-(2-(3-fluoropyridin-4-yl)-11-oxo-11H-pyrido[2,1-b]quinazoline-6-carboxamido)cyclohexane-1-carboxylic acid | - | - | - | A | B |
| 37 | (cis)-4-(11-oxo-2-(pyrimidin-5-yl)-11H-pyrido[2,1-b]quinazoline-6-carboxamido)cyclohexane-1-carboxylic acid | - | - | - | A | B |
| 38 | (cis)-4-(2-(1-methyl-1H-pyrazol-4-yl)-11-oxo-11H-pyrido[2,1-b]quinazoline-6-carboxamido)cyclohexane-1-carboxylic acid | - | - | - | A | A |
| 39 | (cis)-4-(11-oxo-2-(thiophen-2-yl)-11H-pyrido[2,1-b]quinazoline-6-carboxamido)cyclohexane-1-carboxylic acid | - | - | - | A | A |
| 40 | (cis)-4-(8-bromo-11-oxo-11H-pyrido[2,1-b]quinazoline-6-carboxamido)cyclohexane-1-carboxylic acid | - | - | - | B | A |
| 41 | 3-(2-cyclopropyl-11-oxo-11H-pyrido[2,1-b]quinazoline-6-carboxamido)cyclobutane-1-carboxylic acid | - | - | - | B | B |
| 42 | (1S,4S)-4-(2-(5-cyanothiophen-2-yl)-11-oxo-11H-pyrido[2,1-b]quinazoline-6-carboxamido)cyclohexane-1-carboxylic acid | - | - | - | A | A |
| 43 | (1S,4S)-4-(2-(5-carbamoylthiophen-2-yl)-11-oxo-11H-pyrido[2,1-b]quinazoline-6-carboxamido)cyclohexane-1-carboxylic acid | - | - | - | A | A |
| 44 | (1S,4S)-4-(2-(1-methyl-1H-imidazol-4-yl)-11-oxo-11H-pyrido[2,1-b]quinazoline-6-carboxamido)cyclohexane-1-carboxylic acid | - | - | - | A | A |
| 45 | (1S,4S)-4-(2-(5-cyclopropylthiophen-2-yl)-11-oxo-11H-pyrido[2,1-b]quinazoline-6-carboxamido)cyclohexane-1-carboxylic acid | - | - | - | A | A |
| 47 | 5-(6-(((1S,4S)-4-carboxycyclohexyl)carbamoyl)-11-oxo-11H-pyrido[2,1-b]quinazolin-2-yl)thiophene-2-carboxylic acid | - | - | - | A | A |

### Ranges:

A: IC₅₀ < 100 nM
B: 100 nM =< IC₅₀ < 1µM
C: IC₅₀ >= 1 µM

As can be seen from the results described in Table 1, the compounds of the present invention are potent inhibitors of the protein kinases JAK3 and TYK2, showing good selectivity against the enzymes JAK1 and JAK2. Additionally, the compounds above are potent inhibitors of the kinase MLK3.

The derivatives of the invention are useful in the treatment or prevention of diseases known to be susceptible to improvement by treatment with an inhibitor of protein kinase MLK, particularly MLK3 and Janus kinases selected from JAK3 and TYK2. Such diseases are liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer such as gastric, lung, pancreatic, breast, colon, colorectal, and other solid tumours, and others diseases as asthma, chronic obstructive pulmonary disease (COPD), transplant rejection, haematological diseases, uveitis, dry eye and allergic conjunctivitis and neurodegenerative diseases including Alzheimer disease, among others.

Accordingly, the derivatives of the invention and pharmaceutically acceptable salts thereof, and pharmaceutical compositions comprising such compounds and/or salts thereof, may be used in a method of treatment of disorders of the human body which comprises administering to a subject requiring such treatment an effective amount of carboxylic acid derivatives of the present invention or a pharmaceutically acceptable salt thereof.

The present invention also provides pharmaceutical compositions which comprise, as an active ingredient, at least a carboxylic acid derivatives of formula (I) or a pharmaceutically acceptable salt thereof in association with, others therapeutics agents a pharmaceutically acceptable excipient such as a carrier or diluent. The active ingredient may comprise 0.001% to 99% by weight, preferably 0.01% to 90% by weight of the composition depending upon the nature of the formulation and whether further dilution is to be made prior to application.

Preferably, compounds of formula (I), pharmaceutically acceptable salts and compositions thereof are made up in a form suitable for oral, topical, nasal, rectal, percutaneous or injectable administration.

The pharmaceutically acceptable excipients, which are admixed with the active compound or salts of such compound, to form the compositions of this invention, are well known *per se* and the actual excipients used depend inter alia on the intended method of administering the compositions.

Compounds of formula (I), pharmaceutically salts thereof and compositions of this invention are preferably adapted for injectable and per os administration. In this case, the compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents, which may be used in the preparation of the compositions, include those liquid and solid diluents, which are compatible with the active ingredient, together with colouring or flavouring agents, if desired. Tablets or capsules may conveniently contain between 2 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Effective doses are normally in the range of 2-2000 mg of active ingredient per day. Daily dosage may be administered in one or more treatments, preferably from 1 to 4 treatments, per day.

The present invention will be further illustrated by the following examples. The following are given by way of illustration and do not limit the scope of the invention in any way. The synthesis of the compounds of the invention is illustrated by the following examples including the preparation of the intermediates, which do not limit the scope of the invention in any way.

### Abbreviations

In the present application are used the following abbreviations, with the corresponding definitions:
HCl: Hydrochloric acid
HATU:N-[(Dimethylamino)-1H-1,2,3-triazolo-[4,5-b]pyridin-1-ylmethylene]-N-methylmethanaminium hexafluorophosphate N-oxide
EDC: N-(3-Dimethylaminopropyl)-N'-ethylcarbodiimide
HOBt: 1-Hydroxybenzotriazole
T3P: 2,4,6-tripropyl-1,3,5,2,4,6-trioxatriphosphinane 2,4,6-trioxide
EDIA: diisopropylethylamine
DIPEA: N,N-Diisopropylethylamine
THF: tetrahydrofurane
DCM: dichloromethane
DMF: dimethylformamide
CDCl₃: deuterated chloroform
DMSO: dimethylsulfoxide
Pd-Cat: palladium catalyst
Pd(AcO)₂: palladium (II) acetate
R¹-B(OH)₂: boronic acid derivative of R¹
MeOH: methanol
AcOH: acetic acid

### EXAMPLES

**General.** Reagents, solvents and starting products were acquired from commercial sources. The term "concentration" refers to the vacuum evaporation using a Büchi rotavapor. When indicated, the reaction products were purified by "flash" chromatography on silica gel (40-63 µm) with the indicated solvent system. The spectroscopic data were measured in a Varian Mercury 400 spectrometer. The melting points were measured in a Büchi 535 instrument. The HPLC-MS were performed on a Gilson instrument equipped with a Gilson 321 piston pump, a Gilson 864 vacuum degasser, a Gilson 189 injection module, a 1/1000 Gilson splitter, a Gilson 307 pump, a Gilson 170 detector, and a Thermoquest Fennigan aQa detector.

### Intermediate 1: methyl 2-amino-5-cyclopropylbenzoate

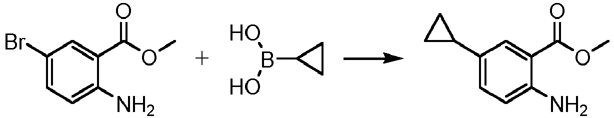

A mixture of methyl 2-amino-5-bromobenzoate (800 mg, 4.18 mmol), cyclopropylboronic acid (776 mg, 10.87 mmol), K₃PO₄ (2.44 g, 14.0 mmol), Pd(AcO)₂ (64 mg, 0.33 mmol) and P(Cy)3 (176 mg, 0.79 mmol) was suspended in toluene (15 mL) and water (0.8 mL) under nitrogen atmosphere and heated for 2 hours at 100ºC. The reaction mixture was filtered through celite and the organic phase was separated, dried and the solvent was removed under reduced pressure, affording 0.65g (yield 81%).
¹H-RMN (400 MHz, CDCl₃): δ = 7.60 (m, 1H), 7.05 (dd, 1H), 6.65 (d, 1H), 1.81 (m, 3H), 0.86 (m, 2H), 0.59 (m, 2H).
HPLC-MS: Rt: 4.656 min, m/z: 192.0 (MH⁺).

The following intermediate were synthesized using the procedure described for the Intermediate 1 from the corresponding boronic acid derivative and 2-amino-5-bromobenzoate.

### Intermediate 2: methyl 4-amino-[1,1'-biphenyl]-3-carboxylate

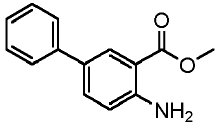

A mixture of methyl 2-amino-5-bromobenzoate (1000 mg, 4.35 mmol), phenylboronic acid (1060 mg, 8.70 mmol), K₃PO₄ (2330 mg, 10.88 mmol), Pd(AcO)₂ (80 mg, 0.35 mmol) and P(Cy)₃ (220 mg, 0.80 mmol) was suspended in toluene (20 mL) and water (1.0 mL) under nitrogen atmosphere and heated for 2 hours at 100ºC. The reaction mixture was filtered through celite and the organic phase was separated, dried and the solvent was removed under reduced pressure, affording 931 mg (yield 95%).
¹H-RMN (400 MHz, CDCl₃): δ = 8.13 (d, 1H), 7.55 (m, 3H), 7.40 (m, 2H), 7.28 (m, 1H), 6.75 (d, 1H), 5.79 (s, 2H), 3.90 (s, 3H).
HPLC-MS: Rt: 5.051 min, m/z: 228.1 (MH⁺).

### Intermediate 3: methyl 4-amino-4'-fluoro-[1,1'-biphenyl]-3-carboxylate

¹H-RMN (400 MHz, CDCl₃): δ = 8.06 (d, 1H), 7.48 (m, 3H), 7.09 (m, 2H), 6.74 (d, 1H), 5.79 (s, 2H), 3.90 (s, 3H).
HPLC-MS: Rt: 5.156 min, m/z: 246.0 (MH⁺).

### Intermediate 4: methyl 4-amino-3'-fluoro-[1,1'-biphenyl]-3-carboxylate

¹H-RMN (400 MHz, CDCl₃): δ = 8.11 (d, 1H), 7.52 (dd, 1H), 7.34 (m, 2H), 7.24 (m, 1H), 6.96 (m, 1H), 6.75 (d, 1H), 5.83 (s, 2H), 3.91 (s, 3H).
HPLC-MS: Rt: 5.113 min, m/z: 245.9 (MH⁺).

### Intermediate 5: methyl 4-amino-3',4'-difluoro-[1,1'-biphenyl]-3-carboxylate

¹H-RMN (400 MHz, CDCl₃): δ = 8.04 (d, 1H), 7.45 (dd, 1H), 7.32 (m, 1H), 7.21 (m, 2H), 6.74 (d, 1H), 5.83 (s, 2H), 3.91 (s, 3H).
HPLC-MS: Rt: 5.250 min, m/z: 263.9 (MH⁺).

### Intermediate 6: methyl 4-amino-4'-cyano-[1,1'-biphenyl]-3-carboxylate

¹H-RMN (400 MHz, CDCl₃): δ = 8.08 (d, 1H), 7.83 (d, 2H), 7.77 (d, 2H), 7.72 (dd, 1H), 6.96 (s, 2H), 6.92 (d, 1H), 3.83 (s, 3H).
HPLC-MS: Rt: 4.745 min, m/z: 252.9 (MH⁺).

### Intermediate 7: methyl 4-amino-3'-cyano-[1,1'-biphenyl]-3-carboxylate

¹H-RMN (400 MHz, CDCl₃): δ = 8.10 (d, 1H), 7.81 (s, 1H), 7.76 (d, 1H), 7.52 (m, 3H), 6.77 (d, 1H), 5.89 (s, 2H), 3.92 (s, 3H).
HPLC-MS: Rt: 4.752 min, m/z: 252.9 (MH⁺).

### Intermediate 8: methyl 2-amino-5-(thiophen-2-yl)benzoate

¹H-RMN (400 MHz, CDCl₃): δ = 8.11 (d, 1H), 7.53 (dd, 1H), 7.18 (m, 2H), 7.04 (dd, 1H), 6.69 (d, 1H), 5.80 (s, 2H), 3.91 (s, 3H).
HPLC-MS: 4.752 min, m/z: 252.9 (MH⁺).

### Intermediate 9: 11-oxo-11H-pyrido[2,1-b]quinazoline-6-carboxylic acid

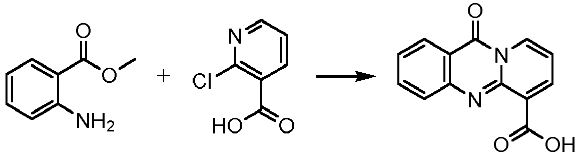

A mixture of methyl 2-aminobenzoate (500 mg, 3.3 mmol), 2-chloronicotinic acid (521 mg, 3.3 mmol) and hydrochloric acid (0.54 mL, 17.8 mmol) in ethanol (8 mL) was stirred at 80ºC for 48 hours. After cooling, the suspension was filtered, washed with cool ethanol and n-pentane and dried. 54% yield.
¹H-RMN (400 MHz, DMSO-d₆): δ = 9.09 (dd, 1H), 8.68 (dd, 1H), 8.35 (dd, 1H), 8.02 (ddd, 1H), 7.90 (m, 1H), 7.63 (ddd, 1H), 7.32 (t, 1H).
HPLC-MS: Rt: 1.100 min, m/z: 240.9 (MH⁺).

### Intermediate 10: methyl 11-oxo-11H-pyrido[2,1-b]quinazoline-6-carboxylate

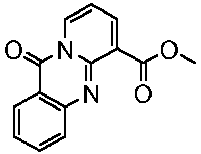

A mixture of 11-oxo-11H-pyrido[2,1-b]quinazoline-6-carboxylic acid (100 mg, 0.416 mmol), sulfuric acid (98%, 0.11 mL, 2.08 mmol) in MeOH (0.3 mL) and MeCN (0.3 mL) was stirred at 85ºC for 20 hours. After cooling, the suspension was added to water (5 mL) and extracted with DCM (2x 5 mL), dried over MgSO₄, filtered through a path of silica gel and the pure product was obtained after removing the solvent under reduced pressure. 61 mg (57% yield).
¹H-RMN (400 MHz, CDCl₃): δ = 8.98 (dd, 1H), 8.44 (d, 1H), 7.85 (m. 3H), 7.51 (m, 1H), 6.86 (t, 1H), 4.04 (s, 3H)
HPLC-MS: Rt: 3.797 min, m/z: 255.0 (MH⁺).

The following intermediate were synthesized using the procedure described for the Intermediate 10 from the corresponding methyl 2-aminobenzoate or 2-aminobenzoic acid and 2-chloronicotinic acid derivatives.

### Intermediate 11: 2-fluoro-11-oxo-11H-pyrido[2,1-b]quinazoline-6-carboxylic acid

¹H-RMN (400 MHz, DMSO-d₆): δ = 9.04 (d, 1H), 8.63 (d, 1H), 8.04 (m, 2H), 7.93 (m, 1H), 7.30 (t, 1H).
HPLC-MS: Rt: 1.35 min, m/z: 259.0 (MH⁺).

### Intermediate 12: 2-chloro-11-oxo-11H-pyrido[2,1-b]quinazoline-6-carboxylic acid

¹H-RMN (400 MHz, DMSO-d₆): δ= 9.06 (dd, 1H), 8.64 (dd, 1H), 8.30 (m, 1H), 8.01 (m, 2H), 7.30 (dd, 1H).
HPLC-MS: Rt: 2.10 min, m/z: 275.0 (MH⁺).

### Intermediate 13: 2-bromo-11-oxo-11H-pyrido[2,1-b]quinazoline-6-carboxylic acid

¹H-RMN (400 MHz, DMSO-d₆): δ = 16.29 (s, 1H), 9.06 (dd, 1H), 8.65 (dd, 1H), 8.42 (m, 1H), 8.12 (dd, 1H), 7.87 (t, 1H), 7.30 (t, 1H).
HPLC-MS: Rt: 1.74 min, m/z: 321.0 (MH⁺).

### Intermediate 14: 2-hydroxy-11-oxo-11H-pyrido[2,1-b]quinazoline-6-carboxylic acid

¹H-RMN (400 MHz, DMSO-d₆): δ = 9.01 (dd, 1H), 8.58 (dd, 1H), 7.82 (t, 1H), 7.61 (d, 1H), 7.52 (dd, 1H), 7.23 (t, 1H), 4.43 (s, 1H).
HPLC-MS: Rt: 5.27 min, m/z: 254.1 (MH⁺).

### Intermediate 15: 2-methoxy-11-oxo-11H-pyrido[2,1-b]quinazoline-6-carboxylic acid

¹H-RMN (400 MHz, DMSO-d₆): δ = 9.03 (dd, 1H), 8.58 (dd, 1H), 7.90 (d, 1H), 7.67 (dd, 1H), 7.53 (m, 1H), 7.25 (t, 1H), 3.94 (s, 3H).
HPLC-MS: Rt: 1.731 min, m/z: 271.0 (MH⁺).

### Intermediate 16: 11-oxo-2-(trifluoromethoxy)-11H-pyrido[2,1-b]quinazoline-6-carboxylic acid

¹H-RMN (400 MHz, DMSO-d₆): δ = 9.05 (dd, 1H), 8.67 (dd, 1H), 8.19 (d, 1H), 8.08 (m, 1H), 7.97 (m, 1H), 7.33 (t, 1H).
HPLC-MS: Rt: 2.854 min, m/z: 325.0 (MH⁺).

### Intermediate 17: 2-methyl-11-oxo-11H-pyrido[2,1-b]quinazoline-6-carboxylic acid

¹H-RMN (400 MHz, DMSO-d₆): δ = 9.03 (dd, 1H), 8.63 (dd, 1H), 8.03 (m, 2H), 7.92 (m, 1H), 7.28 (t, 1H), 2.50 (s, 3H).
HPLC-MS: Rt: 1.27 min, m/z: 259.0 (MH⁺).

### Intermediate 18: 2-cyclopropyl-11-oxo-11H-pyrido[2,1-b]quinazoline-6-carboxylic acid

¹H-RMN (400 MHz, DMSO-d₆): δ = 9.02 (dd, 1H), 8.60 (dd, 1H), 8.00 (m, 1H), 7.78 (d, 1H), 7.69 (dd, 2H), 7.25 (t, 1H), 2.19 (m, 2H), 1.08 (m, 2H), 0.82 (m, 2H).
HPLC-MS: Rt: 2.36 min, m/z: 281.1 (MH⁺).

### Intermediate 19: 2-cyclopropyl-8-methyl-11-oxo-11H-pyrido[2,1-b]quinazoline-6-carboxylic acid

¹H-RMN (400 MHz, DMSO-d₆): δ = 8.85 (dd, 1H), 8.50 (d, 1H), 7.99 (d, 1H), 7.77 (d, 1H), 7.67 (dd, 1H), 2.42 (s, 3H), 2.18 (m, 1H), 1.08 (m, 2H), 0.82 (m, 2H)
HPLC-MS: Rt: 2.917 min, m/z: 295.0 (MH⁺).

### Intermediate 20: 8-methyl-11-oxo-11H-pyrido[2,1-b]quinazoline-6-carboxylic acid

¹H-RMN (400 MHz, DMSO-d₆): δ= 8.89 (m, 1H), 8.56 (d, 1H), 8.34 (dd, 1H), 7.99 (m, 1H), 7.87 (d, 1H), 7.60 (m, 1H), 2.43 (s, 3H).
HPLC-MS: Rt: 1.944 min, m/z: 255.0 (MH⁺).

### Intermediate 21: 11-oxo-2-phenyl-11H-pyrido[2,1-b]quinazoline-6-carboxylic acid

¹H-RMN (400 MHz, DMSO-d₆): δ = 9.07 (dd, 1H), 8.64 (dd, 1H), 8.51 (d, 1H), 8.32 (dd, 1H), 7.97 (d, 1H), 7.82 (m, 2H), 7.53 (t, 2H), 7.44 (m, 1H), 7.29 (t, 1H).

### Intermediate 22: 2-(4-fluorophenyl)-11-oxo-11H-pyrido[2,1-b]quinazoline-6-carboxylic acid

¹H-RMN (400 MHz, DMSO-d₆): δ = 9.09 (dd, 1H), 8.65 (dd, 1H), 8.54 (d, 1H), 8.34 (dd, 1H), 8.01 (d, 1H), 7.91 (m, 2H), 7.37 (m, 2H), 7.30 (t, 1H).
HPLC-MS: Rt: 3.157 min, m/z: 335.0 (MH⁺).

### Intermediate 23: 2-(3-fluorophenyl)-11-oxo-11H-pyrido[2,1-b]quinazoline-6-carboxylic acid

¹H-RMN (400 MHz, DMSO-d₆): δ = 9.10 (d, 1H), 8.67 (d, 1H), 8.59 (s, 1H), 8.38 (d, 1H), 8.01 (d, 1H), 7.72 (m, 2H), 7.58 (dd, 1H), 7.29 (m, 2H)
HPLC-MS: Rt: 3.227 min, m/z: 334.8 (MH⁺).

### Intermediate 24: 2-(3,4-fluorophenyl)-11-oxo-11H-pyrido[2,1-b]quinazoline-6-carboxylic acid

¹H-RMN (400 MHz, DMSO-d₆): δ = 9.10 (d, 1H), 8.67 (d, 1H), 8.59 (d, 1H), 8.36 (dd, 1H), 8.01 (m, 2H), 7.73 (m, 1H), 7.59 (dd, 1H), 7.31 (t, 1H)
HPLC-MS: Rt: 3.357 min, m/z: 352.8 (MH⁺).

### Intermediate 25: 2-(4-cyanophenyl)-11-oxo-11H-pyrido[2,1-b]quinazoline-6-carboxylic acid

¹H-RMN (400 MHz, DMSO-d₆): δ = 9.11 (d, 1H), 8.67 (m, 2H), 8.42 (d, 1H), 8.07 (dd, 3H), 7.99 (d, 2H), 7.32 (t, 1H).
HPLC-MS: Rt: 3.016 min, m/z: 341.8 (MH⁺).

### Intermediate 26: 2-(3-cyanophenyl)-11-oxo-11H-pyrido[2,1-b]quinazoline-6-carboxylic

**acid** ¹H-RMN (400 MHz, DMSO-d₆): δ = 9.10 (dd, 1H), 8.66 (m, 2H), 8.40 (m, 2H), 8.21 (d, 1H), 8.02 (d, 1H), 7.88 (d, 1H), 7.73 (t, 1H), 7.31 (t, 1H)
HPLC-MS: Rt: 3.043 min, m/z: 341.8 (MH⁺).

### Intermediate 27: 11-oxo-2-(thiophen-2-yl)-11H-pyrido[2,1-b]quinazoline-6-carboxylic acid

¹H-RMN (400 MHz, DMSO-d₆): δ = 13.78 (s, 1H), 9.07 (dd, 1H), 8.64 (dd, 1H), 8.42 (d, 1H), 8.34 (dd, 1H), 7.97 (d, 1H), 7.77 (d, 1H), 7.67 (d, 1H), 7.30 (t, 1H), 7.22 (dd, 1H).
HPLC-MS: Rt: 3.083 min, m/z: 323.0 (MH⁺).

### Intermediate 28: methyl 4-(2-cyclopropyl-11-oxo-11H-pyrido[2,1-b]quinazoline-6-carboxamido)cyclohexane-1-carboxylate

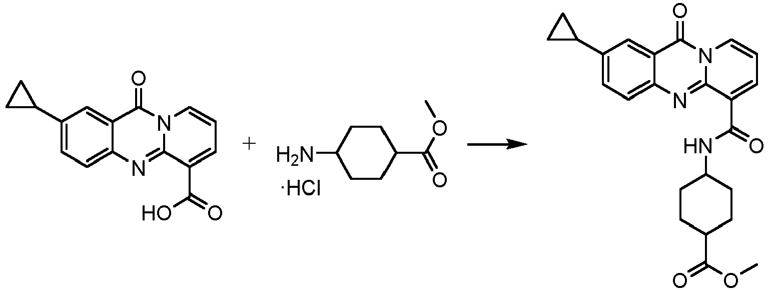

A mixture of 2-cyclopropyl-11-oxo-11H-pyrido[2,1-b]quinazoline-6-carboxylic acid (50 mg, 0.18 mmol), HATU (77 mg, 0.20 mmol) and DIPEA (0.14 mL, 0.79 mmol) in DMF (1 mL) was stirred 10 min at room temperature. Then methyl 4-aminocyclohexane-1-carboxylate hydrochloride (70 mg, 0.36 mmol) was added and the mixture was stirred 18 hours at room temperature. The product was precipitated in cool water, filtered and washed with water and pentane. 66 mg (77% yield).
¹H-RMN (400 MHz, CDCl₃): δ = 11.53 (m, 1H), 11.29 (m, 1H), 9.04 (dd, 2H), 8.77 (dd, 2H), 8.09 (m, 2H), 7.84 (d, 1H), 7.62 (m, 3H), 7.00 (m, 2H), 4.44 (m, 1H), 4.02 (m, 1H), 3.74 (s, 3H), 3.70 (s, 3H), 2.54 (m, 1H), 2.39 (dt, 1H), 2.29 (m, 2H), 2.09 (m, 2H), 1.97 (m, 4H), 1.72 (m, 8H), 1.48 (m, 4H), 1.10 (m, 4H), 0.85 (m, 4H).
HPLC-MS: Rt: 5.374 min, m/z: 420.0 (MH⁺).

The following intermediate were synthesized using the procedure described for the Intermediate 28 from the corresponding 11-oxo-11H-pyrido[2,1-b]quinazoline-6-carboxylic acid derivative and amine.

### Intermediate 29: methyl (1s,4s)-4-(2-cyclopropyl-11-oxo-11H-pyrido[2,1-b]quinazoline-6-carboxamido)cyclohexane-1-carboxylate

¹H-RMN (400 MHz, CDCl₃): δ = 11.53 (d, 1H), 9.04 (dd, 1H), 8.79 (dd, 1H), 8.11 (d, 1H), 7.84 (d, 1H), 7.65 (dd, 1H), 7.01 (m, 1H), 4.44 (m, 1H), 3.75 (s, 3H), 2.53 (m, 1H), 2.08 (m, 1H), 1.98 (m, 6H), 1.80 (m, 2H), 1.10 (m, 2H), 0.85 (dt, 2H).
HPLC-MS: Rt: 5.404 min, m/z: 420.0 (MH⁺).

### Intermediate 30: methyl (1r,4r)-4-(2-cyclopropyl-11-oxo-11H-pyrido[2,1-b]quinazoline-6-carboxamido)cyclohexane-1-carboxylate

¹H-RMN (400 MHz, CDCl₃): δ = 11.28 (d, 1H), 9.03 (d, 1H), 8.77 (d, 1H), 8.09 (d, 1H), 7.62 (dt, 2H), 7.00 (m, 1H), 4.02 (m, 1H), 3.70 (s, 3H), 2.40 (m, 1H), 2.29 (m, 2H), 2.10 (m, 3H), 1.57 (m, 4H), 1.11 (m, 2H), 0.85 (m, 2H).
HPLC-MS: Rt: 5.477 min, m/z: 420.0 (MH⁺).

### Intermediate 31: methyl 4-(2-cyclopropyl-11-oxo-11H-pyrido[2,1-b]quinazoline-6-carboxamido)bicyclo[2.2.2]octane-1-carboxylate

¹H-RMN (400 MHz, CDCl₃): δ = 11.38 (s, 1H), 9.01 (dd, 1H), 8.72 (dd, 1H), 8.08 (d, 1H), 7.61 (m, 2H), 6.99 (t, 1H), 3.68 (s, 3H), 2.18 (m, 6H), 2.08 (m, 1H), 1.99 (m, 6H), 1.11 (m, 2H), 0.85 (m, 2H)
HPLC-MS: Rt: 5.893 min, m/z: 446.2 (MH⁺).

### Intermediate 32: methyl 4-(2-cyclopropyl-11-oxo-11H-pyrido[2,1-b]quinazoline-6-carboxamido)bicyclo[2.2.1]heptane-1-carboxylate

¹H-RMN (400 MHz, CDCl₃): δ = 11.74 (s, 1H), 9.02 (dd, 1H), 8.74 (dd, 1H), 8.08 (d, 1H), 7.59 (m, 2H), 7.00 (t, 1H), 3.72 (s, 3H), 2.14 (m, 11H), 1.10 (m, 2H), 0.85 (m, 2H)
HPLC-MS: Rt: 5.697 min, m/z: 432.2 (MH⁺).

### Intermediate 33: methyl 1-(2-cyclopropyl-11-oxo-11H-pyrido[2,1-b]quinazoline-6-carboxamido)cyclopentane-1-carboxylate

¹H-RMN (400 MHz, CDCl₃): δ = 11.89 (s, 1H), 9.03 (dd,1H), 8.72 (dd, 1H), 8.09 (d, 1H), 7.63 (m, 2H), 6.98 (t, 1H), 3.78 (s, 3H), 2.39 (m, 2H), 2.26 (m, 2H), 2.09 (m, 1H), 1.94 (m, 4H), 1.11 (m, 2H), 0.85 (m, 2H)
HPLC-MS: Rt: 5.563 min, m/z: 406.1 (MH⁺).

### Intermediate 34: methyl 2-(2-cyclopropyl-11-oxo-11H-pyrido[2,1-b]quinazoline-6-carboxamido)-2-methylpropanoate

¹H-RMN (400 MHz, CDCl₃): δ = 12.01 (s, 1H), 9.02 (dd, 1H), 8.73 (dd, 1H), 8.09 (d, 1H), 7.74 (dd, 1H), 7.63 (dd, 1H), 7.00 (t, 1H), 3.81 (s, 3H), 2.08 (m, 1H), 1.77 (s, 6H), 1.10 (m, 2H), 0.85 (m, 2H).
HPLC-MS: Rt: 5.289 min, m/z: 380.1 (MH⁺).

### Intermediate 35: methyl (1s,4s)-4-(2-cyclopropyl-8-methyl-11-oxo-11H-pyrido[2,1-b]quinazoline-6-carboxamido)cyclohexane-1-carboxylate

¹H-RMN (400 MHz, CDCl₃): δ = 11.61 (d, 1H), 8.84 (s, 1H), 8.65 (d, 1H), 8.10 (d, 1H), 7.82 (d, 1H), 7.63 (dd 1H), 4.44 (m, 1H), 3.74 (s, 3H), 2.53 (m, 1H), 2.42 (s, 3H), 2.09 (m, 1H), 1.96 (m, 6H), 1.81 (m, 2H), 1.09 (m, 2H), 0.85 (m, 2H).
HPLC-MS: Rt: 5.710 min, m/z: 434.2 (MH⁺).

### Intermediate 36: methyl(1s,4s)-4-(2-cyclopentyl-11-oxo-11H-pyrido[2,1-b]quinazoline-6-carboxamido)cyclohexane-1-carboxylate

¹H-RMN (400 MHz, CDCl₃): δ = 11.56 (d, 1H), 9.05 dd, 1H), 8.77 (dd, 1H), 8.26 (d, 1H), 7.84 (m, 2H), 7.00 (t, 1H), 4.45 (m, 1H), 3.74 (s, 3H), 3.21 (m, 1H), 2.53 (m, 1H), 2.15 (m, 2H), 1.80 (m, 14H).
HPLC-MS: Rt: 5.814 min, m/z: 448.2 (MH⁺).

### Intermediate 37: methyl 4-(8-methyl-11-oxo-11H-pyrido[2,1-b]quinazoline-6-carboxamido) cyclohexane-1-carboxylate

¹H-RMN (400 MHz, CDCl₃): δ = 11.60 (d, 1H), 11.37 (d, 1H), 8.86 (m, 2H), 8.70 (dd, 2H), 8.45 (d, 2H), 7.89 (m, 3H), 7.68 (d, 1H), 7.52 (m, 2H), 4.45 (m, 1H), 4.02 (m, 1H), 3.74 (s, 3H), 3.70 (s, 3H), 2.54 (m, 1H), 2.43 (s, 6H), 2.35 (m, 3H), 2.12 (m, 2H), 2.00 (m, 7H), 1.74 (m, 5H).
HPLC-MS: Rt: 5.045 min, m/z: 394.1 (MH⁺).

### Intermediate 38: methyl(cis)-4-(8-methyl-11-oxo-11H-pyrido[2,1-b]quinazoline-6-carboxamido)cyclohexane-1-carboxylate

¹H-RMN (400 MHz, CDCl₃): δ = 11.59 (d, 1H), 8.86 (dd, 1H), 8.70 (d, 1H), 8.45 (d, 1H), 7.90 (m, 2H), 7.52 (m, 1H), 4.43 (m, 1H), 3.73 (s, 3H), 2.54 (m, 1H), 2.43 (s, 3H), 1.98 (m, 6H), 1.80 (m, 2H)

HPLC-MS: Rt: 5.097 min, m/z: 394.1 (MH⁺).

### Intermediate 39: methyl 4-(11-oxo-11H-pyrido[2,1-b]quinazoline-6-carboxamido) cyclohexane-1-carboxylate

¹H-RMN (400 MHz, CDCl₃): δ = 11.53 (d, 1H), 11.29 (d, 1H), 9.06 (ddd, 2H), 8.88 (d, 1H), 8.83 (dd, 1H), 8.46 (m, 2H), 7.91 (m, 3H), 7.70 (dd, 1H), 7.54 (m, 2H), 7.05 (dt, 2H), 4.44 (m, 1H), 4.02 (dt, 1H), 3.74 (s, 3H), 3.71 (s, 3H), 2.54 (m, 1H), 2.40 (ddd, 1H), 2.30 (m, 2H), 2.12 (m, 2H), 1.99 (m, 4H), 1.74 (m, 8H), 1.48 (m, 4H).
HPLC-MS: Rt: 4.706 min, m/z: 380.0 (MH⁺).

### Intermediate 40: methyl (cis)-4-(11-oxo-11H-pyrido[2,1-b]quinazoline-6-carboxamido)cyclohexane-1-carboxylate

¹H-RMN (400 MHz, CDCl₃): δ = 11.53 (d, 1H), 9.06 (dd, 1H), 8.82 (dd, 1H), 8.46 (dd, 1H), 7.92 (m, 2H), 7.54 (m, 1H), 7.03 (t, 1H), 4.44 (m, 1H), 3.74 (s, 3H), 2.55 (m, 1H), 1.92 (m, 8H)
HPLC-MS: Rt: 4.758 min, m/z: 380.1 (MH⁺).

### Intermediate 41: methyl 4-(2-hydroxy-11-oxo-11H-pyrido[2,1-b]quinazoline-6-carboxamido) cyclohexane-1-carboxylate

¹H-RMN (400 MHz, DMSO-d₆): δ = 11.25 (d, 1H), 11.03 (d, 1H), 10.29 (d, 2H), 8.92 (m, 2H), 8.49 (m, 2H), 7.73 (m, 2H), 7.58 (m, 2H), 7.49 (dd, 2H), 7.12 (m, 2H), 4.24 (m, 1H), 3.81 (m, 1H), 3.67 (s, 3H), 3.62 (s, 3H), 2.50 (m, 2H), 2.10 (m, 2H), 1.98 (m, 2H), 1.81 (m, 8H), 1.51 (m, 4H).
HPLC-MS: Rt: 4.121 min, m/z: 369.1 (MH⁺).

### Intermediate 42: methyl 4-(2-methoxy-11-oxo-11H-pyrido[2,1-b]quinazoline-6-carboxamido) cyclohexane-1-carboxylate

¹H-RMN (400 MHz, CDCl₃): δ = 11.48 (d, 1H), 11.24 (d, 1H), 9.03 (m, 2H), 8.74 (m, 2H), 7.87 (d, 1H), 7.73 (m, 2H), 7.63 (d, 1H), 7.52 (m, 2H), 7.01 (m, 2H), 4.43 (m, 1H), 4.02 (m, 1H), 3.96 (s, 6H), 3.73 (s, 3H), 3.69 (s, 3H), 2.53 (m, 1H), 2.39 (m, 1H), 2.30 (m, 2H), 2.11 (m, 2H), 1.96 (m, 6H), 1.72 (m, 8H).
HPLC-MS: Rt: 4.901 y 5.245 min, m/z: 410.1 (MH⁺).

### Intermediate 43: methyl (1s,4s)-4-(11-oxo-2-(trifluoromethoxy)-11H-pyrido[2,1-b]quinazoline-6-carboxamido)cyclohexane-1-carboxylate

¹H-RMN (400 MHz, CDCl₃): δ = 11.32 (d, 1H), 9.05 (dd, 1H), 8.85 (dd, 1H), 8.27 (s, 1H), 8.03 (dd, 1H), 7.76 (dd, 1H), 7.09 (t, 1H), 4.46 (m, 1H), 3.74 (s, 3H), 2.54 (m, 1H), 1.95 (m, 6H), 1.80 (m, 2H).
HPLC-MS: Rt: 5.549 min, m/z: 463.8 (MH⁺).

### Intermediate 44: methyl 4-(2-cyano-11-oxo-11H-pyrido[2,1-b]quinazoline-6-carboxamido) cyclohexane-1-carboxylate

¹H-RMN (400 MHz, CDCl₃): δ = 11.22 (d, 1H), 9.09 (m, 1H), 8.96 (m, 1H), 8.79 (m, 1H), 8.07 (m, 2H), 7.18 (m, 1H), 4.47 (m, 1H), 3.75 (s, 3H), 2.55 (m, 1H), 2.30 (m, 1H), 2.12 (m, 1H), 1.75 (m, 6H).
HPLC-MS: Rt: 4.657 min, m/z: 405.1 (MH⁺).

### Intermediate 45: methyl 4-(2-fluoro-11-oxo-11H-pyrido[2,1-b]quinazoline-6-carboxamido) cyclohexane-1-carboxylate

¹H-RMN (400 MHz, CDCl₃): δ = 11.37 (d, 1H), 11.10 (d, 1H), 9.02 (m, 2H), 8.83 (m, 2H), 8.07 (m, 2H), 8.00 (dd, 1H), 7.67 (m, 3H), 7.06 (m, 2H), 4.45 (m, 1H), 4.02 (m, 1H), 3.74 (s, 3H), 3.70 (s, 3H), 2.54 (m, 1H), 2.40 (m, 1H), 2.30 (m, 2H), 2.12 (m, 2H), 1.96 (m, 4H), 1.61 (m, 8H).
HPLC-MS: Rt: 4.940 min, m/z: 398.1 (MH⁺).

### Intermediate 46: methyl 4-(2-chloro-11-oxo-11H-pyrido[2,1-b]quinazoline-6-carboxamido) cyclohexane-1-carboxylate

¹H-RMN (400 MHz, CDCl₃): δ =11.36 (d, 1H), 11.08 (d, 1H), 9.03 (m, 2H), 8.84 (m, 2H), 8.40 (m, 2H), 7.93 (d, 1H), 7.84 (dd, 1H), 7.80 (dd, 1H), 7.63 (d, 1H), 7.07 (m, 2H), 4.45 (m, 1H), 4.02 (m, 1H), 3.74 (s, 3H), 3.70 (s, 3H), 2.54 (m, 1H), 2.39 (m, 1H), 2.30 (m, 2H), 2.12 (m, 2H), 1.95 (m, 5H), 1.72 (m, 7H).
HPLC-MS: Rt: 5.243 min, m/z: 414.1 (MH⁺).

### Intermediate 47: methyl 4-(2-bromo-11-oxo-11H-pyrido[2,1-b]quinazoline-6-carboxamido) cyclohexane-1-carboxylate

¹H-RMN (400 MHz, CDCl₃): δ = 11.35 (d, 1H), 11.07 (d, 1H), 9.04 (m, 2H), 8.85 (m, 2H), 8.57 (m, 2H), 7.95 (m, 2H), 7.86 (d, 1H), 7.55 (d, 1H), 7.07 (m, 2H), 4.45 (m, 1H), 4.01 (m, 1H), 3.74 (s, 3H), 3.70 (s, 3H), 2.54 (m, 1H), 2.40 (m, 1H), 2.30 (m, 2H), 2.12 (m, 2H), 1.95 (m, 6H), 1.59 (m, 6H).
HPLC-MS: Rt: 5.350 min, m/z: 460.0 (M⁺2H⁺).

### Intermediate 48: methyl (1s,4s)-4-(2-bromo-11-oxo-11H-pyrido[2,1-b]quinazoline-6-carboxamido)cyclohexane-1-carboxylate

¹H-RMN (400 MHz, CDCl₃): δ = 11.35 (d, 1H), 9.04 (m, 1H), 8.85 (m, 1H), 8.57 (d, 1H), 7.98 (m, 1H), 7.85 (d, 1H), 7.07 (t, 1H), 4.45 (m, 1H), 3.74 (s, 3H), 2.53 (m, 1H), 1.94 (m, 5H), 1.80 (m, 3H).
HPLC-MS: Rt: 5.316 min, m/z: 460.1 (MH⁺).

### Intermediate 49: methyl(1s,4s)-4-(11-oxo-2-phenyl-11H-pyrido[2,1-b]quinazoline-6-carboxamido)cyclohexane-1-carboxylate

¹H-RMN (400 MHz, CDCl₃): δ = 11.55 (m, 1H), 9.08 (dd, 1H), 8.83 (m, 1H), 8.68 (d, 1H), 8.20 (dd, 1H), 8.02 (d, 1H), 7.75 (dd, 2H), 7.51 (t, 2H), 7.42 (m, 1H), 7.05 (dd, 1H), 4.48 (m, 1H), 3.77 (s, 3H), 2.56 (m, 1H), 1.98 (m, 6H), 1.84 (m, 2H).
HPLC-MS: Rt: 5.758 min, m/z: 456.1 (MH⁺).

### Intermediate 50: methyl(1s,4s)-4-(2-(4-fluorophenyl)-11-oxo-11H-pyrido[2,1-b]quinazoline-6-carboxamido)cyclohexane-1-carboxylate

¹H-RMN (400 MHz, CDCl₃): δ = 11.53 (d, 1H), 9.08 (dd, 1H), 8.83 (dd, 1H), 8.62 (d, 1H), 8.14 (dd, 1H), 8.03 (d, 1H), 7.70 (m, 2H), 7.20 (m, 2H), 7.06 (t, 1H), 4.47 (m, 1H), 3.75 (s, 3H), 2.56 (m, 1H), 1.99 (m, 6H), 1.83 (m, 2H).
HPLC-MS: Rt: 5.720 min, m/z: 474.2 (MH⁺).

### Intermediate 51: methyl(1s,4s)-4-(11-oxo-2-(pyridin-4-yl)-11H-pyrido[2,1-b]quinazoline-6-carboxamido)cyclohexane-1-carboxylate

¹H-RMN (400 MHz, CDCl₃): δ = 11.46 (d, 1H), 9.10 (dd, 1H), 8.87 (dd, 1H), 8.75 (m, 3H), 8.22 (dd, 1H), 8.10 (d, 1H), 7.67 (d, 2H), 7.09 (t, 1H), 4.48 (m, 1H), 3.76 (s, 3H), 2.56 (m, 1H), 1.98 (m, 6H), 1.83 (m, 2H).
HPLC-MS: Rt: 4.678 min, m/z: 457.2 (MH⁺).

### Intermediate 52: methyl(1s,4s)-4-(2-morpholino-11-oxo-11H-pyrido[2,1-b]quinazoline-6-carboxamido)cyclohexane-1-carboxylate

¹H-RMN (400 MHz, CDCl₃): δ = 11.35 (d, 1H), 9.03 (dd, 1H), 8.72 (dd, 1H), 7.91 (d, 1H), 7.72 (d, 1H), 7.64 (dd, 1H), 6.98 (t, 1H), 4.45 (m, 1H), 3.93 (m, 4H), 3.74 (s, 3H), 3.33 (m, 4H), 2.54 (m, 1H), 1.94 (m, 4H), 1.78 (m, 4H).
HPLC-MS: Rt: 4.717 min, m/z: 465.2 (MH⁺).

### Intermediate 53: methyl 3-(11-oxo-11H-pyrido[2,1-b]quinazoline-6-carboxamido) cyclohexane-1-carboxylate

¹H-RMN (400 MHz, CDCl₃): δ = 11.34 (d, 1H), 9.05 (dd, 1H), 8.84 (m, 1H), 8.45 (m, 1H), 7.90 (m, 1H), 7.71 (m, 1H), 7.53 (m, 1H), 7.03 (t, 1H), 4.07 (m, 1H), 3.68 (s, 3H), 2.51 (m, 1H), 2.20 (m, 1H), 1.89 (m, 5H), 1.45 (m, 2H).
HPLC-MS: Rt: 4.831 min, m/z: 380.1 (MH⁺).

### Intermediate 54: methyl(1R,4R)-4-(8-methyl-11-oxo-11H-pyrido[2,1-b]quinazoline-6-carboxamido)cyclohexane-1-carboxylate

¹H-RMN (400 MHz, CDCl₃): δ = 11.35 (d, 1H), 8.85 (s, 1H), 8.69 (d, 1H), 8.45 (d, 1H), 7.87 (m, 1H), 7.68 (d, 1H), 7.51 (t, 1H), 4.03 (m, 1H), 3.71 (s, 3H), 2.43 (s, 3H), 2.39 (m, 1H), 2.31 (m, 2H), 2.13 (m, 2H), 1.70 (m, 2H), 1.46 (m, 2H).
HPLC-MS: Rt: 5.192 min, m/z: 393.9 (MH⁺).

### Intermediate 55: methyl 3-(2-cyclopropyl-11-oxo-11H-pyrido[2,1-b]quinazoline-6-carboxamido)cyclobutane-1-carboxylate

¹H-RMN (400 MHz, CDCl₃) Major diastereoisomer δ = 11.63 (d, 1H), 9.03 (d, 1H), 8.73 (d, 1H), 8.10 (d, 1H), 7.72 (d, 1H), 7.65 (m, 1H), 6.98 (t, 1H), 4.62 (m, 1H), 3.74 (s, 3H), 2.98 (m, 1H), 2.83 (m, 2H), 2.42 (m, 2H), 2.10 (m, 1H), 1.11 (m, 2H), 0.86 (m, 2H).
HPLC-MS: Rt: 4.971 min, m/z: 391.8 (MH⁺).

### Intermediate 56: methyl trans-4-(2-bromo-11-oxo-11H-pyrido[2,1-b]quinazoline-6-carboxamido)cyclohexane-1-carboxylate

¹H-RMN (400 MHz, CDCl₃) δ = 11.35 (d, 1H), 9.04 (m, 1H), 8.85 (m, 1H), 8.57 (d, 1H), 7.98 (m, 1H), 7.85 (d, 1H), 7.07 (t, 1H), 4.45 (m, 1H), 3.74 (s, 3H), 2.53 (m, 1H), 1.94 (m, 5H), 1.80 (m, 3H).
HPLC-MS: Rt: 5.290 min, m/z: 457.7 (MH⁺).

### Intermediate 57: methyl (trans)-4-(2-(4-fluorophenyl)-11-oxo-11H-pyrido[2,1-b]quinazoline-6-carboxamido)cyclohexane-1-carboxylate

¹H-RMN (400 MHz, CDCl₃): δ = 11.24 (d, 1H), 9.07 (dd, 1H), 8.81 (dd, 1H), 8.61 (d, 1H), 8.09 (dd, 1H), 7.76 (d, 1H), 7.69 (m, 2H), 7.20 (t, 2H), 7.05 (t, 1H), 4.04 (m, 1H), 3.71 (s, 3H), 2.42 (m, 1H), 2.32 (m, 2H), 2.13 (m, 2H), 1.69 (m, 2H), 1.48 (m, 2H).
HPLC-MS: Rt: 5.674 min, m/z: 473.8 (MH⁺).

### Intermediate 58: methyl (cis)-4-(2-(3-fluorophenyl)-11-oxo-11H-pyrido[2,1-b]quinazoline-6-carboxamido)cyclohexane-1-carboxylate

¹H-RMN (400 MHz, CDCl₃): δ = 11.50 (d, 1H), 9.09 (d, 1H), 8.84 (d, 1H), 8.66 (d, 1H), 8.16 (dd, 1H), 8.04 (d, 1H), 7.48 (m, 3H), 7.08 (m, 2H), 4.47 (m, 1H), 3.77 (s, 3H), 2.56 (m, 1H), 1.99 (m, 6H), 1.84 (m, 2H).
HPLC-MS: Rt: 5.681 min, m/z: 473.8 (MH⁺).

### Intermediate 59: methyl(cis)-4-(2-(3,4-difluorophenyl)-11-oxo-11H-pyrido[2,1-b]quinazoline-6-carboxamido)cyclohexane-1-carboxylate

¹H-RMN (400 MHz, CDCl₃): δ = 11.47 (d, 1H), 9.09 (dd, 1H), 8.83 (dd, 1H), 8.60 (d, 1H), 8.09 (m, 2H), 7.55 (m, 1H), 7.46 (m, 1H), 7.31 (m, 1H), 7.07 (t, 1H), 4.47 (m, 1H), 3.76 (s, 3H), 2.55 (m, 1H), 1.99 (m, 6H), 1.84 (m, 2H).
HPLC-MS: Rt: 5.839 min, m/z: 491.8 (MH⁺).

### Intermediate 60: methyl (cis)-4-(2-(4-cyanophenyl)-11-oxo-11H-pyrido[2,1-b]quinazoline-6-carboxamido)cyclohexane-1-carboxylate

¹H-RMN (400 MHz, DMSO-d₆): δ = 11.21 (d, 1H), 9.07 (d, 1H), 8.66 (dd, 2H), 8.39 (dd, 1H), 8.09 (d, 2H), 7.97 (dd, 3H), 7.25 (t, 1H), 4.28 (m, 1H), 2.58 (m, 1H), 3.70 (s, 3H), 1.85 (m, 8H).
HPLC-MS: Rt: 5.421 min, m/z: 480.8 (MH⁺).

### Intermediate 61: methyl (cis)-4-(2-(3-cyanophenyl)-11-oxo-11H-pyrido[2,1-b]quinazoline-6-carboxamido)cyclohexane-1-carboxylate

¹H-RMN (400 MHz, CDCl₃): δ = 11.47 (d, 1H), 9.10 (dd, 1H), 8.87 (dd, 1H), 8.66 (d, 1H), 8.13 (m, 2H), 7.99 (m, 2H), 7.70 (d, 1H), 7.63 (t, 1H), 7.09 (t, 1H), 4.48 (m, 1H), 3.76 (s, 3H), 2.56 (m, 1H), 1.99 (m, 6H), 1.84 (m, 2H).
HPLC-MS: Rt: 5.324 min, m/z: 480.8 (MH⁺).

### Intermediate 62: methyl 4-(6-(((cis)-4-(methoxycarbonyl)cyclohexyl)carbamoyl)-11-oxo-11H-pyrido[2,1-b]quinazolin-2-yl)benzoate

¹H-RMN (400 MHz, CDCl₃): δ = 11.49 (d, 1H), 9.10 (dd, 1H), 8.85 (dd, 1H), 8.72 (d, 1H), 8.22 (dd, 1H), 8.17 (d, 2H), 8.06 (d, 1H), 7.82 (d, 2H), 7.07 (t, 1H), 4.47 (m, 1H), 3.97 (s, 3H), 3.75 (s, 3H), 2.56 (m, 1H), 2.00 (m, 6H), 1.82 (m, 2H).
HPLC-MS: Rt: 5.604 min, m/z: 513.8 (MH⁺).

### Intermediate 63: methyl (cis)-4-(11-oxo-2-(pyridin-3-yl)-11H-pyrido[2,1-b]quinazoline-6-carboxamido)cyclohexane-1-carboxylate

¹H-RMN (400 MHz, DMSO-d₆): δ = 11.18 (d, 1H), 9.02 (m, 2H), 8.63 (d, 2H), 8.55 (d, 1H), 8.33 (dd, 1H), 8.25 (d, 1H), 7.88 (d, 1H), 7.54 (dd, 1H), 7.22 (t, 1H), 4.26 (m, 1H), 3.70 (s, 3H), 2.57 (m, 1H), 1.83 (m, 8H).
HPLC-MS: Rt: 4.608 min, m/z: 456.9 (MH⁺).

### Intermediate 64: methyl (trans)-4-(11-oxo-2-(pyridin-4-yl)-11H-pyrido[2,1-b]quinazoline-6-carboxamido)cyclohexane-1-carboxylate

¹H-RMN (400 MHz, CDCl₃): δ = 11.18 (d, 1H), 9.09 (d, 1H), 8.86 (d, 1H), 8.75 (m, 3H), 8.17 (d, 1H), 7.81 (d, 1H), 7.66 (m, 2H), 7.09 (t, 1H), 4.05 (m, 1H), 3.71 (s, 3H), 2.42 (m, 1H), 2.33 (m, 2H), 2.14 (m, 2H), 1.67 (m, 4H).
HPLC-MS: Rt: 4.605 min, m/z: 456.8 (MH⁺).

### Intermediate 65: methyl (cis)-4-(2-(3-fluoropyridin-4-yl)-11-oxo-11H-pyrido[2,1-b]quinazoline-6-carboxamido)cyclohexane-1-carboxylate

¹H-RMN (400 MHz, CDCl₃): δ = 11.44 (d, 1H), 9.10 (dd, 1H), 8.88 (dd, 1H), 8.73 (s, 1H), 8.58 (m, 2H), 8.19 (d, 1H), 8.08 (d, 1H), 7.56 (m, 1H), 7.10 (t, 1H), 4.47 (m, 1H), 3.75 (s, 3H), 2.56 (m, 1H), 1.99 (m, 6H), 1.82 (m, 2H).
HPLC-MS: Rt: 4.801 min, m/z: 474.8 (MH⁺).

### Intermediate 66: methyl (cis)-4-(11-oxo-2-(pyrimidin-5-yl)-11H-pyrido[2,1-b]quinazoline-6-carboxamido)cyclohexane-1-carboxylate

¹H-RMN (400 MHz, DMSO-d₆): δ= 11.18 (s, 1H), 9.31 (s, 2H), 9.23 (s, 1H), 9.04 (d, 1H), 8.66 (d, 2H), 8.41 (d, 1H), 7.92 (d, 1H), 7.25 (d, 1H), 4.28 (m, 1H), 2.57 (m, 1H), 3.70 (s, 3H), 1.84 (m, 8H).
HPLC-MS: Rt: 4.251 min, m/z: 457.9 (MH⁺).

### Intermediate 67: methyl (cis)-4-(2-(1-methyl-1H-pyrazol-4-yl)-11-oxo-11H-pyrido[2,1-b]quinazoline-6-carboxamido)cyclohexane-1-carboxylate

¹H-RMN (400 MHz, DMSO-d₆): δ = 11.24 (d, 1H), 9.00 (dd, 1H), 8.59 (dd, 1H), 8.41 (s, 1H), 8.39 (s, 1H), 8.19 (dd, 1H), 8.06 (s, 1H), 7.81 (d, 1H), 7.18 (t, 1H), 4.27 (m, 1H), 3.90 (s, 3H), 3.69 (s, 3H), 2.56 (m, 1H), 1.83 (m, 8H).
HPLC-MS: Rt: 3.272 min, m/z: 443.7 (MH⁺).

### Intermediate 68: methyl (cis)-4-(11-oxo-2-(thiophen-2-yl)-11H-pyrido[2,1-b]quinazoline-6-carboxamido)cyclohexane-1-carboxylate

¹H-RMN (400 MHz, DMSO-d₆): δ = 11.47 (d, 1H), 9.07 (dd, 1H), 8.82 (dd, 1H), 8.65 (d, 1H), 8.18 (dd, 1H), 7.96 (d, 1H), 7.50 (dd, 1H), 7.37 (d, 1H), 7.15, (dd, 1H), 7.05 (t, 1H), 4.46 (m, 1H), 3.76 (s, 3H), 2.55 (m, 1H), 1.99 (m, 6H), 1.81 (m, 2H).
HPLC-MS: Rt: 5.757 min, m/z: 462.1 (MH⁺).

### Intermediate 69: methyl (1s,4s)-4-(2-(5-methylthiophen-2-yl)-11-oxo-11H-pyrido[2,1-b]quinazoline-6-carboxamido)cyclohexane-1-carboxylate

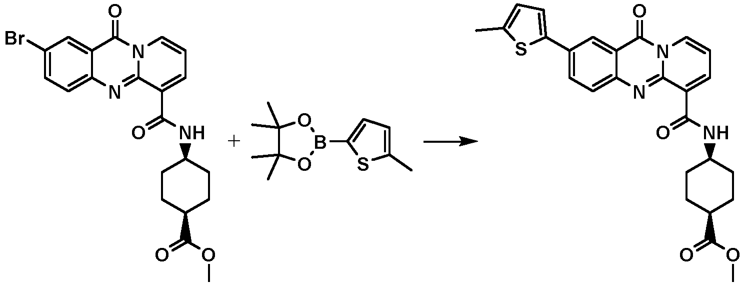

In a screw cap vial was added methyl (1s,4s)-4-(2-bromo-11-oxo-11H-pyrido[2,1-b]quinazoline-6-carboxamido)cyclohexane-1-carboxylate (0.150 g, 0.33 mmol, 1.0 eq.), 4,4,5,5-tetramethyl-2-(5-methylthiophen-2-yl)-1,3,2-dioxaborolane (0.088 g, 0.39 mmol, 1.2 eq), bis(triphenylphosphine)palladium(II) dichloride (0.023 g, 0.032 mmol, 0.1 eq), potassium carbonate (0.091 g, 0.65 mmol, 2 eq) was disolved in 2.1 ml of dioxane/wáter (3:1) and purged with argón. The resultant mixture was heating under orbital stirring at 110ºC for 2 hours and then was cooled at room temperature and the solvent was removed under reduced pressure, being the resulting residue purified by flash column chromatography on silica gel using dichloromethane/methanol (1%) as eluent to afford methyl (1s,4s)-4-(2-(5-methylthiophen-2-yl)-11-oxo-11H-pyrido[2,1-b]quinazoline-6-carboxamido)cyclohexane-1-carboxylate as a yellow solid (0.102 g, 66%).
¹H-RMN (300 MHz, CDCl₃): δ =11.47 (d, *J* = 7.0 Hz, 1H), 9.04 (dd, *J* = 7.3, 1.8 Hz, 1H), 8.78 (d, *J* = 7.0 Hz, 1H), 8.53 (d, *J* = 2.2 Hz, 1H), 8.08 (dd, *J* = 8.7, 2.2 Hz, 1H), 7.92 (s, 1H), 7.28 (s, 1H), 7.02 (t, *J* = 7.1 Hz, 1H), 6.77 (d, *J* = 4.7 Hz, 1H), 4.45 (bs, 1H), 3.75 (s, 3H), 2.54 (s, 4H), 2.08 - 1.88 (m, 6H), 1.88-1.70 (m, 2H).

The following intermediate were synthesized using the procedure described for the Intermediate 69 from the corresponding 4,4,5,5-tetramethyl-1,3,2-dioxaborolane or boronic acid derivative and methyl (1s,4s)-4-(2-bromo-11-oxo-11H-pyrido[2,1-b]quinazoline-6-carboxamido)cyclohexane-1-carboxylate.

### Intermediate 70: methyl (1s,4s)-4-(2-(1-methyl-1H-imidazol-4-yl)-11-oxo-11H-pyrido[2,1-b]quinazoline-6-carboxamido)cyclohexane-1-carboxylate

¹H-RMN (300 MHz, CDCl₃): δ = 11.51 (d, *J* = 9.5 Hz, 1H), 9.06 (d, *J* = 7.3 Hz, 1H), 8.79 (d, *J* = 7.0 Hz, 1H), 8.50 (s, 1H), 8.04 (dd, *J* = 8.7, 2.1 Hz, 1H), 7.98 - 7.89 (m, 2H), 7.79 (s, 1H), 7.03 (t, *J* = 7.0 Hz, 1H), 4.46 (bs, 1H), 3.99 (s, 3H), 3.75 (s, 3H), 2.55 (bs, 1H), 2.14 - 1.87 (m, 6H), 1.87 - 1.67 (m, 2H).

### Intermediate 71: methyl (1s,4s)-4-(2-(5-cyanothiophen-2-yl)-11-oxo-11H-pyrido[2,1-b]quinazoline-6-carboxamido)cyclohexane-1-carboxylate

¹H-RMN (300 MHz, CDCl₃): δ = 11.11 (d, *J* = 9.5 Hz, 1H), 9.02 (d, *J* = 8.7 Hz, 1H), 8.65 (d, *J* = 9.5 Hz, 1H), 8.53 (s, 1H), 8.34 (d, *J* = 12.6 Hz, 1H), 8.05 (d, *J* = 2.4 Hz, 1H), 7.96 - 7.83 (m, 2H), 7.25 (t, *J* = 7.2 Hz, 1H), 4.26 (bs, 1H), 3.69 (s, 3H), 2.57 (bs, 1H), 1.96 - 1.65 (m, 8H).

### Intermediate 72: methyl 5-(6-(((1s,4s)-4-(methoxycarbonyl)cyclohexyl)carbamoyl)-11-oxo-11H-pyrido[2,1-b]quinazolin-2-yl)thiophene-2-carboxylate

¹H-RMN (300 MHz, CDCl₃): δ = 11.43 (d, *J* = 9.2 Hz, 1H), 9.08 (d, *J* = 7.3 Hz, 1H), 8.85 (d, *J* = 8.7 Hz, 1H), 8.70 (s, 1H), 8.17 (d, *J* = 8.7 Hz, 1H), 8.01 (d, *J* = 8.8 Hz, 1H), 7.82 (d, *J* = 3.9 Hz, 1H), 7.48 (d, *J* = 3.9 Hz, 1H), 7.08 (t, *J* = 7.3 Hz, 1H), 4.47 (bs, 1H), 3.93 (s, 3H), 3.76 (s, 3H), 2.56 (bs, 1H), 1.98 (d, *J* = 5.5 Hz, 6H), 1.83 (d, *J* = 10.0 Hz, 2H).

### Intermediate 73: methyl (1s,4s)-4-(11-oxo-2-(tributylstannyl)-11H-pyrido[2,1-b]quinazoline-6-carboxamido)cyclohexane-1-carboxylate

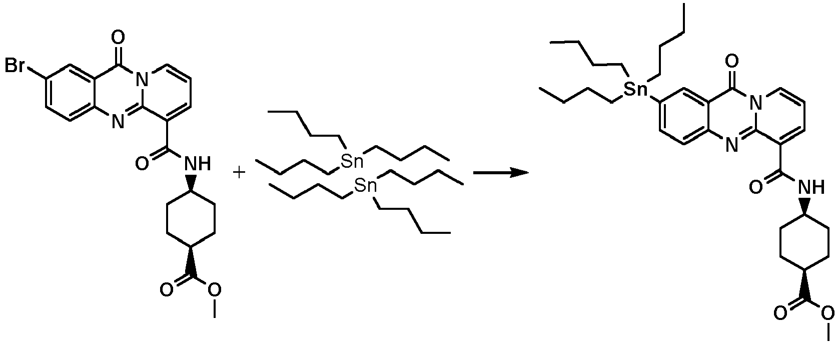

In a screw cap vial was added methyl (1s,4s)-4-(2-bromo-11-oxo-11H-pyrido[2,1-b]quinazoline-6-carboxamido)cyclohexane-1-carboxylate (0.70 g, 1.53 mmol, 1.0 eq.), bis(dibenzylideneacetone)palladium(0) (0.063 g, 0.069 mmol, 0.045 eq), tricyclohexylphosphine (0.043 g, 0.153 mmol, 0.1 eq) was disolved in 20 ml of dry dioxane. Then, 1,1,1,2,2,2-hexabutyldistannane (2.08 ml, 4.12 mmol, 2.7 eq) was added dropwise and purged with argon. The resultant mixture was heating under orbital stirring at 110ºC for 8 hours and then was cooled at room temperature and filtered over celite and washed with ethyl acetate and dichloromethane. The solvent was removed under reduced pressure, being the resulting residue purified by flash column chromatography on silica gel using hexane/ethylacetate (2:1) as eluent to afford methyl methyl (1s,4s)-4-(11-oxo-2-(tributylstannyl)-11H-pyrido[2,1-b]quinazoline-6-carboxamido)cyclohexane-1-carboxylate as a yellow oil (0.790 g, 64%).
¹H-RMN (300 MHz, CDCl₃): δ = 11.58 (d, *J* = 7.4 Hz, 1H), 9.07 (dd, *J* = 7.3, 1.7 Hz, 1H), 8.80 (dd, *J* = 7.0, 1.7 Hz, 1H), 8.54 (s, 1H), 8.01 (d, *J* = 8.1 Hz, 1H), 7.85 (d, *J* = 8.1 Hz, 1H), 7.01 (t, *J* = 7.1 Hz, 1H), 4.43 (s, 1H), 3.74 (s, 3H), 2.54 (s, 1H), 2.03 - 1.91 (m, 4H), 1.86 - 1.76 (m, 2H), 1.65 - 1.48 (m, 6H), 1.42 - 1.26 (m, 7H), 1.23 - 1.10 (m, 5H), 0.89 (t, *J* = 7.3 Hz, 9H).

### Intermediate 74: methyl (1s,4s)-4-(2-(5-cyclopropylthiophen-2-yl)-11-oxo-11H-pyrido[2,1-b]quinazoline-6-carboxamido)cyclohexane-1-carboxylate

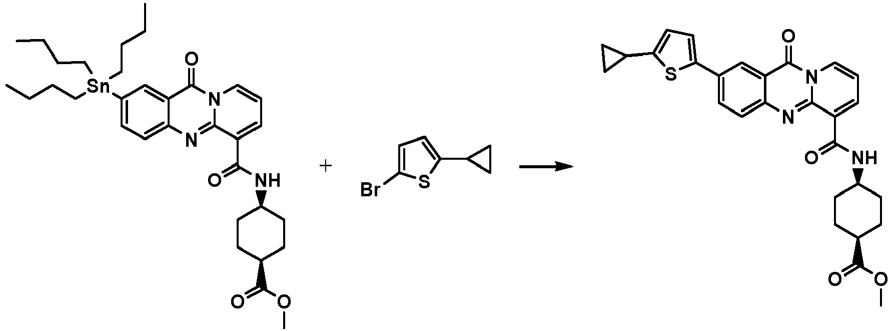

In a screw cap vial was added methyl (1s,4s)-4-(11-oxo-2-(tributylstannyl)-11H-pyrido[2,1-b]quinazoline-6-carboxamido)cyclohexane-1-carboxylate (0.062 g, 0.93 mmol, 1.1 eq.), 2-bromo-5-cyclopropylthiophene (0.011 ml, 0.084 mmol, 1 eq), palladium acetate (0.002 g, 0.008 mmol, 0.1 eq), tricyclohexylphosphine (0.005 g, 0.017 mmol, 0.2 eq), Cesium fluoride (0.019 g, 0.126 mmol, 1.5 eq) was disolved in 0.5 ml of THF. The resultant mixture was heating under orbital stirring at 90ºC for 60 minutes and then was cooled at room temperature and the solvent was removed under reduced pressure, being the resulting residue purified by flash column chromatography on silica gel using dichloromethane/metahnol (1%) as eluent to afford methyl (1s,4s)-4-(2-(5-cyclopropylthiophen-2-yl)-11-oxo-11H-pyrido[2,1-b]quinazoline-6-carboxamido)cyclohexane-1-carboxylate as a yellow solid (0.030 g, 65%).
¹H-RMN (300 MHz, CDCl₃): δ = 11.49 (d, *J* = 10.4 Hz, 1H), 9.05 (d, *J* = 7.7 Hz, 1H), 8.79 (d, *J* = 7.0 Hz, 1H), 8.55 (s, 1H), 8.10 (d, *J* = 5.8 Hz, 1H), 7.92 (d, *J* = 9.6 Hz, 1H), 7.28 (d, *J* = 5.5 Hz, 1H), 7.03 (t, *J* = 7.7 Hz, 1H), 6.79 (d, *J* = 5.5 Hz, 1H), 4.46 (s, 1H), 3.76 (s, 3H), 2.56 (s, 1H), 2.21 - 2.07 (m, 1H), 1.97 (t, *J* = 8.0 Hz, 6H), 1.82 (d, *J* = 10.9 Hz, 2H), 1.06 (dd, *J* = 8.3, 2.3 Hz, 2H), 0.93 - 0.73 (m, 2H).

The following intermediate were synthesized using the procedure described for the Intermediate 74 from the corresponding bromo derivative and methyl (1s,4s)-4-(11-oxo-2-(tributylstannyl)-11H-pyrido[2,1-b]quinazoline-6-carboxamido)cyclohexane-1-carboxylate.

### Intermediate 75: methyl (1s,4s)-4-(11-oxo-2-(thiazol-5-yl)-11H-pyrido[2,1-b]quinazoline-6-carboxamido)cyclohexane-1-carboxylate

¹H-RMN (300 MHz, CDCl₃): δ = 11.39 (d, *J* = 7.6 Hz, 1H), 9.13 - 8.98 (m, 1H), 8.82 (d, *J* = 4.4 Hz, 2H), 8.57 (d, *J* = 2.0 Hz, 1H), 8.23 (s, 1H), 8.11 (dd, *J* = 8.7, 2.0 Hz, 1H), 7.99 (d, *J* = 8.7 Hz, 1H), 7.06 (t, *J* = 7.6 Hz, 1H), 4.44 (s, 1H), 3.75 (s, 3H), 2.55 (s, 1H), 2.02 - 1.79 (m, 8H).

### EXAMPLES

### Example 1: 4-(2-cyclopropyl-11-oxo-11H-pyrido[2,1-b]quinazoline-6-carboxamido)cyclohexane-1-carboxylic acid

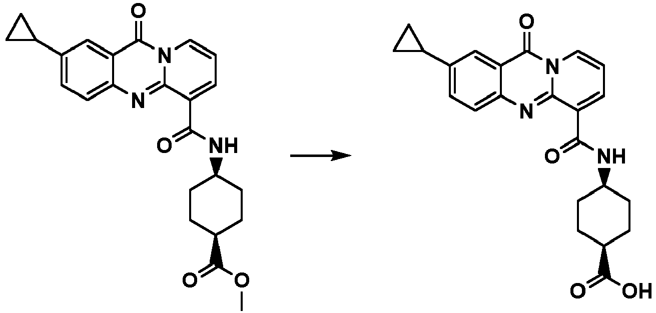

Method A: To a solution of methyl 4-(2-cyclopropyl-11-oxo-11H-pyrido[2,1-b]quinazoline-6-carboxamido)cyclohexane-1-carboxylate (62 mg, 0.15 mmol) in THF (0.75 mL) NaOH 1N (0.75 mL, 0.75 mmol) was added at room temperature and the mixture was stirred for 2 hours. The product was isolated as a pale yellow solid after acidification (pH 4-5) with 2N HCl, followed by filtration and purification through flash column chromatography (DCM:MeOH 95:5). 73% yield.
¹H-RMN (400 MHz, DMSO-d₆): δ = 12.24 (s, 1H), 11.28 (d, 1H), 8.98 (dd, 1H), 8.58 (dd, 1H), 8.00 (d, 1H), 7.79 (d, 1H), 7.60 (m, 1H), 7.16 (t, 1H), 4.26 (m, 1H), 2.44 (m, 1H), 2.18 (m, 1H), 1.81 (m, 8H), 1.07 (m, 2H), 0.80 (m, 2H).
HPLC-MS: Rt: 3.225 min and 3.403 min; m/z: 406.1 (MH⁺).

### Example 2: (1S,4S)-4-(2-(5-methylthiophen-2-yl)-11-oxo-11H-pyrido[2,1-b]quinazoline-6-carboxamido)cyclohexane-1-carboxylic acid

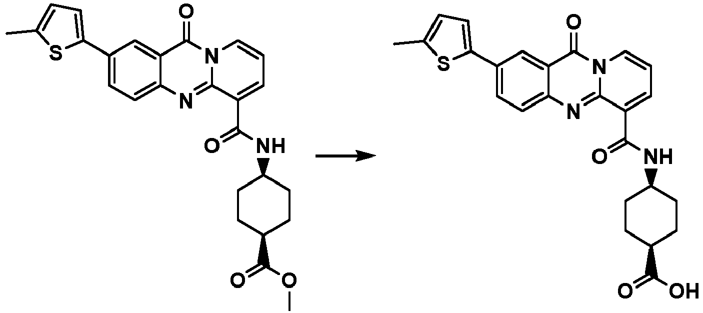

Method B: In a screw cap vial was added methyl (1s,4s)-4-(2-(5-methylthiophen-2-yl)-11-oxo-11H-pyrido[2,1-b]quinazoline-6-carboxamido)cyclohexane-1-carboxylate (0.50 g, 0.11 mmol, 1.0 eq.), lithium hydroxide monohydrate (0.013 g, 0.52 mmol, 5 eq) was suspended in 4 ml of THF/water (4:1). The resultant mixture was heating under orbital stirring at 50ºC for 3 hours and then was cooled at room temperature and the solvent was removed under reduced pressure, adjust pH= 4 with HCl (2N). The mixture was washed with ethyl acetate, dichloromethane and the mixture was dried (Na₂SO₄). The solvent was removed under reduced pressure, being the resulting residue purified by flash column chromatography on silica gel using dichloromethane/methanol (2%) as eluent to afford (1s,4s)-4-(2-(5-methylthiophen-2-yl)-11-oxo-11H-pyrido[2,1-b]quinazoline-6-carboxamido)cyclohexane-1-carboxylic acid as a yellow solid (0.032 g, 64%).
¹H-RMN (300 MHz, DMSO-d₆): 12.32 (s, 1H), 11.19 (d, *J* = 8.7 Hz, 1H), 8.95 (d, *J* = 8.7 Hz, 1H), 8.57 (d, *J* = 8.4 Hz, 1H), 8.26 (s, 1H), 8.09 (d, *J* = 10.6 Hz, 1H), 7.84 (d, *J* = 8.6 Hz, 1H), 7.43 (d, *J* = 3.3 Hz, 1H), 7.17 (t, *J* = 7.1 Hz, 1H), 6.92 - 6.76 (m, 1H), 4.25 (bs, 1H), 2.49 (s, 3H), 2.40 (bs, 1H), 1.97-1.56 (m, 8H
HPLC-MS: Rt: 10.63 min, m/z: 462.1 (MH⁺)

The following intermediate were synthesized using the procedure described for the example 1 or 2 from the corresponding methyl 4-(11-oxo-11H-pyrido[2,1-b]quinazoline-6-carboxamido)carboxylate derivative.

### Example 3: (1S,4S)-4-(2-cyclopropyl-11-oxo-11H-pyrido[2,1-b]quinazoline-6-carboxamido)cyclohexane-1-carboxylic acid

¹H-RMN (400 MHz, DMSO-d₆): δ= 12.24 (s, 1H), 11.28 (d, 1H), 8.98 (dd, 1H), 8.58 (dd, 1H), 8.00 (d, 1H), 7.79 (d, 1H), 7.60 (dd, 1H), 7.16 (t, 1H), 4.26 (m, 1H), 2.44 (m, 1H), 2.18 (m, 1H), 1.81 (m, 8H), 1.07 (m, 2H), 0.80 (m, 2H).
HPLC-MS: Rt: 3.288 min, m/z: 406.0 (MH⁺).

### Example 4: (1R,4R)-4-(2-cyclopropyl-11-oxo-11H-pyrido[2,1-b]quinazoline-6-carboxamido)cyclohexane-1-carboxylic acid

¹H-RMN (400 MHz, DMSO-d₆): δ = 12.12 (s, 1H), 11.02 (d, 1H), 8.97 (dd, 1H), 8.55 (dd, 1H), 7.98 (s, 1H), 7.69 (m, 2H), 7.16 (t, 1H), 3.81 (m, 1H), 2.33 (m, 1H), 2.18 (m, 1H), 2.08 (m, 2H), 1.99 (m, 2H), 1.51 (m, 4H), 1.08 (m, 2H), 0.82 (m, 2H).
HPLC-MS: Rt: 3.480 min, m/z: 406.0 (MH⁺).

### Example 5: (1S,4S)-4-(11-oxo-2-(trifluoromethoxy)-11H-pyrido[2,1-b]quinazoline-6-carboxamido)cyclohexane-1-carboxylic acid

¹H-RMN (400 MHz, DMSO-d₆): δ = 12.28 (s, 1H), 11.07 (d, 1H), 9.00 (dd, 1H), 8.66 (dd, 1H), 8.16 (s, 1H), 8.02 (d, 1H), 7.89 (dd, 1H), 7.26 (t, 1H), 4.26 (m, 1H), 2.44 (m, 1H), 1.83 (m, 8H).
HPLC-MS: Rt: 3.413 min, m/z: 450.1 (MH⁺).

### Example 6: 4-(2-cyclopropyl-11-oxo-11H-pyrido[2,1-b]quinazoline-6-carboxamido)bicyclo[2.2.2]octane-1-carboxylic acid

¹H-RMN (400 MHz, DMSO-d₆): δ = 1.23 (s, 1H), 11.15 (s, 1H), 8.93 (dd, 1H), 8.52 (dd, 1H), 7.94 (d, 1H), 7.66 (dd, 1H), 7.58 (d, 1H), 7.13 (t, 1H), 2.16 (m, 1H), 2.06 (m, 6H), 1.86 (m, 6H), 1.06 (m, 2H), 0.79 (m, 2H).
HPLC-MS: Rt: 3.619 min, m/z: 432.2 (MH⁺).

### Example 7: 4-(2-cyclopropyl-11-oxo-11H-pyrido[2,1-b]quinazoline-6-carboxamido)bicyclo[2.2.1]heptane-1-carboxylic acid

¹H-RMN (400 MHz, DMSO-d₆): δ = 11.44 (s, 1H), 8.95 (dd, 1H), 8.55 (dd, 1H), 7.95 (d, 1H), 7.65 (dd, 1H), 7.59 (d, 1H), 7.16 (t, 1H), 2.15 (m, 3H), 2.08 (s, 2H), 2.03 (m, 2H), 1.90 (t, 2H), 1.70 (t, 2H), 1.07 (m, 2H), 0.80 (m, 2H).
HPLC-MS: Rt: 3.545 min, m/z: 418.1 (MH⁺).

### Example 8: 1-(2-cyclopropyl-11-oxo-11H-pyrido[2,1-b]quinazoline-6-carboxamido)cyclopentane-1-carboxylic acid

¹H-RMN (400 MHz, DMSO-d₆): δ = 12.53 (s, 1H), 11.64 (s, 1H), 8.98 (dd, 1H), 8.55 (dd, 1H), 7.99 (s, 1H), 7.69 (m, 2H), 7.16 (t, 1H), 2.19 (m, 5H), 1.84 (m, 4H), 1.08 (m, 2H), 0.81 (m, 2H).
HPLC-MS: Rt: 3.555 min, m/z: 392.1 (MH⁺).

### Example 9: 2-(2-cyclopropyl-11-oxo-11H-pyrido[2,1-b]quinazoline-6-carboxamido)-2-methylpropanoic acid

¹H-RMN (400 MHz, DMSO-d₆): δ = 12.83 (s, 1H), 11.87 (s, 1H), 8.99 (d, 1H), 8.58 (d, 1H), 7.98 (dd, 1H), 7.79 (d, 1H), 7.69 (dd, 1H), 7.17 (t, 1H), 0.88 (m, 1H), 1.65 (s, 6H), 1.08 (m, 2H), 0.82 (m, 2H).
HPLC-MS: Rt: 3.271 min, m/z: 366.1 (MH⁺).

### Example 10: 4-(8-methyl-11-oxo-11H-pyrido[2,1-b]quinazoline-6-carboxamido)cyclohexane-1-carboxylic acid

¹H-RMN (400 MHz, DMSO-d₆): δ = 12.23 (s, 2H), 11.33 (d, 1H), 11.07 (d, 1H), 8.83 (d, 2H), 8.52 (d, 1H), 8.47 (d, 1H), 8.32 (m, 2H), 7.92 (m, 3H), 7.79 (d, 1H), 7.56 (m, 2H), 4.27 (m, 1H), 3.82 (m, 1H), 2.40 (s, 6H), 2.33 (m, 2H), 2.10 (m, 2H), 1.98 (m, 2H), 1.84 (m, 8H), 1.50 (m, 4H).
HPLC-MS: Rt: 2.986 / 3.135 min, m/z: 380.1 (MH⁺).

### Example 11: (1S,4S)-4-(8-methyl-11-oxo-11H-pyrido[2,1-b]quinazoline-6-carboxamido)cyclohexane-1-carboxylic acid

¹H-RMN (400 MHz, DMSO-d6): δ = 11.33 (d, 1H), 8.81 (s, 1H), 8.50 (d, 1H), 8.30 (m, 1H), 7.90 (m, 2H), 7.55 (t, 1H), 4.26 (m, 1H), 2.46 (m, 1H), 2.39 (s, 3H), 1.80 (m, 8H).
HPLC-MS: Rt: 3.034 min, m/z: 380.1 (MH⁺).

### Example 12: 4-(11-oxo-11H-pyrido[2,1-b]quinazoline-6-carboxamido)cyclohexane-1-carboxylic acid

¹H-RMN (400 MHz, DMSO-d₆): δ = 12.21 (s, 2H), 11.30 (d, 1H), 11.05 (d, 1H), 8.99 (td, 2H), 8.61 (ddd, 2H), 8.32 (m, 2H), 7.94 (ddt, 3H), 7.79 (d, 1H), 7.58 (m, 2H), 7.19 (td, 2H), 4.27 (m, 1H), 3.82 (m, 1H), 2.44 (m, 1H), 2.33 (m, 1H), 2.09 (m, 2H), 1.99 (m, 2H), 1.82 (m, 6H), 1.50 (m, 4H).
HPLC-MS: Rt: 2.865 / 3.015 min, m/z: 366.0 (MH⁺).

### Example 13: (1S,4S)-4-(11-oxo-11H-pyrido[2,1-b]quinazoline-6-carboxamido)cyclohexane-1-carboxylic acid

¹H-RMN (400 MHz, DMSO-d₆): δ = 12.30 (s, 1H), 11.30 (d, 1H), 9.00 (dd, 1H), 8.63 (dd, 1H), 8.32 (d, 1H), 7.91 (m, 2H), 7.57 (dd, 1H), 7.20 (t, 1H), 4.27 (m, 1H), 2.40 (m, 1H), 1.82 (m, 8H)
HPLC-MS: Rt: 2.833 min, m/z: 366.1 (MH⁺).

### Example 14: (1S,4S)-4-(2-morpholino-11-oxo-11H-pyrido[2,1-b]quinazoline-6-carboxamido)cyclohexane-1-carboxylic acid

¹H-RMN (400 MHz, DMSO-d₆): δ = 12.26 (s, 1H), 11.28 (d, 1H), 8.93 (d, 1H), 8.50 (d, 1H), 7.79 (m, 2H), 7.53 (s, 1H), 7.13 (t, 1H), 4.24 (m, 1H), 3.80 (m, 4H), 3.26 (m, 4H), 1.77 (m, 9H).
HPLC-MS: Rt: 2.923 min, m/z: 451.2 (MH⁺).

### Example 15: (1S,4S)-4-(11-oxo-2-phenyl-11H-pyrido[2,1-b]quinazoline-6-carboxamido)cyclohexane-1-carboxylic acid

¹H-RMN (400 MHz, DMSO-d₆): δ = 11.26 (d, 1H), 9.03 (dd, 1H), 8.63 (dd, 1H), 8.51 (d, 1H), 8.25 (dd, 1H), 7.97 (d, 1H), 7.82 (m, 2H), 7.54 (t, 2H), 7.44 (t, 1H), 7.22 (t, 1H), 4.27 (m, 1H), 2.46 (m, 1H), 1.83 (m, 8H).
HPLC-MS: Rt: 3.521 min, m/z: 442.1 (MH⁺).

### Example 16: (1S,4S)-4-(2-(4-fluorophenyl)-11-oxo-11H-pyrido[2,1-b]quinazoline-6-carboxamido)cyclohexane-1-carboxylic acid

¹H-RMN (400 MHz, DMSO-d₆): δ = 11.25 (d, 1H), 9.01 (dd, 1H), 8.62 (dd, 1H), 8.47 (d, 1H), 8.21 (dd, 1H), 7.94 (d, 1H), 7.85 (m, 2H), 7.36 (m, 2H), 7.21 (t, 1H), 4.26 (m, 1H), 2.45 (m, 1H), 1.85 (m, 8H).
HPLC-MS: Rt: 3.569 min, m/z: 460.1 (MH⁺).

### Example 17: (1S,4S)-4-(11-oxo-2-(pyridin-4-yl)-11H-pyrido[2,1-b]quinazoline-6-carboxamido)cyclohexane-1-carboxylic acid

¹H-RMN (400 MHz, DMSO-d₆): δ = 11.24 (d, 1H), 9.06 (dd, 1H), 8.72 (m, 3H), 8.68 (dd, 1H), 8.38 (dd, 1H), 8.02 (d, 1H), 7.94 (d, 2H), 7.26 (t, 1H), 4.27 (m, 1H), 2.33 (m, 1H), 1.85 (m, 8H).
HPLC-MS: Rt: 3.084 min, m/z: 443.1 (MH⁺).

### Example 18: (1S,4S)-4-(2-cyclopropyl-8-methyl-11-oxo-11H-pyrido[2,1-b]quinazoline-6-carboxamido)cyclohexane-1-carboxylic acid

¹H-RMN (400 MHz, DMSO-d₆): δ = 12.21 (s, 1H), 11.31 (d, 1H), 8.81 (s, 1H), 8.47 (s, 1H), 8.01 (s, 1H), 7.79 (d, 1H), 7.60 (d, 1H), 4.26 (m, 1H), 2.44 (m, 1H), 2.39 (s, 3H), 2.19 (m, 1H), 1.80 (m, 8H), 1.64 (m, 2H), 0.81 (m, 2H).
HPLC-MS: Rt: 3.471 min, m/z: 420.1 (MH⁺).

### Example 19: 4-(2-hydroxy-11-oxo-11H-pyrido[2,1-b]quinazoline-6-carboxamido)cyclohexane-1-carboxylic acid

¹H-RMN (400 MHz, DMSO-d₆): δ= 11.29 (d, 1H), 11.03 (d, 1H), 8.91 (t, 2H), 8.54 (m, 1H), 8.47 (d, 1H), 7.83 (d, 1H), 7.71 (d, 1H), 7.56 (t, 2H), 7.49 (dd, 1H), 7.44 (dd, 1H), 7.11 (m, 2H), 4.26 (m, 1H), 3.80 (m, 2H), 2.41 (m, 1H), 2.33 (m, 1H), 2.08 (m, 2H), 1.97 (m, 2H), 1.80 (m, 8H), 1.51 (m, 4H).
HPLC-MS: Rt: 2.656 min, m/z: 382.1 (MH⁺).

### Example 20: 4-(2-methoxy-11-oxo-11H-pyrido[2,1-b]quinazoline-6-carboxamido)cyclohexane-1-carboxylic acid

¹H-RMN (400 MHz, DMSO-d₆): δ = 12.24 (s, 1H), 11.22 (d, 1H), 8.97 (dd, 1H), 8.55 (dd, 1H), 7.85 (d, 1H), 7.61 (m, 1H), 7.53 (dd, 1H), 7.16 (m, 1H), 4.26 (m, 1H), 3.92 (s, 1H), 2.33 (m, 1H), 2.08 (m, 1H), 1.98 (m, 1H), 1.81 (m, 4H), 1.48 (m, 2H).
HPLC-MS: Rt: 2.941 min, m/z: 396.1 (MH⁺).

### Example 21: 4-(2-cyano-11-oxo-11H-pyrido[2,1-b]quinazoline-6-carboxamido)cyclohexane-1-carboxylic acid

¹H-RMN (400 MHz, DMSO-d₆): δ = 12.24 (s, 1H), 11.00 (d, 1H), 9.05 (m, 1H), 8.73 (m, 2H), 8.19 (dd, 1H), 7.93 (m, 1H), 7.32 (m, 1H), 4.25 (m, 1H), 2.45 (m, 1H), 1.99 (m, 1H), 1.81 (m, 7H).
HPLC-MS: Rt: 2.895 min, m/z: 391.1 (MH⁺).

### Example 22: 4-(2-fluoro-11-oxo-11H-pyrido[2,1-b]quinazoline-6-carboxamido)cyclohexane-1-carboxylic acid

¹H-RMN (400 MHz, DMSO-d₆): δ= 12.27 (s, 1H), 11.12 (d, 1H), 8.97 (m, 1H), 8.60 (dd, 1H), 7.99 (m, 2H), 7.82 (m, 1H), 7.21 (dd, 1H), 4.26 (m, 1H), 2.44 (m, 1H), 1.96 (m, 2H), 1.81 (m, 6H).
HPLC-MS: Rt: 2.920 / 3.081 min, m/z: 384.1 (MH⁺).

### Example 23: 4-(2-chloro-11-oxo-11H-pyrido[2,1-b]quinazoline-6-carboxamido)cyclohexane-1-carboxylic acid

¹H-RMN (400 MHz, DMSO-d₆): δ= 12.28 (s, 1H), 11.10 (d, 1H), 9.01 (m, 1H), 8.65 (dd, 1H), 8.28 (m, 1H), 7.92 (m, 2H), 7.24 (t, 1H), 4.25 (m, 1H), 2.45 (m, 1H), 1.99 (m, 1H), 1.80 (m, 7H).
HPLC-MS: Rt: 3.111 / 3.308 min, m/z: 400.1 (MH⁺).

### Example 24: 4-(2-bromo-11-oxo-11H-pyrido[2,1-b]quinazoline-6-carboxamido)cyclohexane-1-carboxylic acid

¹H-RMN (400 MHz, DMSO-d₆): δ = 12.20 (s, 1H), 11.09 (d, 1H), 9.01 (dd, 1H), 8.65 (dd, 1H), 8.41 (d, 1H), 8.03 (dd, 1H), 7.84 (d, 1H), 7.24 (t, 1H), 4.25 (m, 1H), 2.43 (m, 1H), 2.15 (m, 1H), 1.94 (m, 2H), 1.80 (m, 6H).
HPLC-MS: Rt: 3.136 min, m/z: 444.0 (MH⁺).

### Example 25: 3-(11-oxo-11H-pyrido[2,1-b]quinazoline-6-carboxamido)cyclohexane-1-carboxylic acid

¹H-RMN (400 MHz, DMSO-d₆): δ = 12.18 (s, 1H), 11.03 (d, 1H), 8.99 (dd, 1H), 8.59 (dd, 1H), 8.31 (dd, 1H), 7.98 (m, 1H), 7.80 (d, 1H), 7.58 (m, 1H), 7.18 (t, 1H), 3.89 (m, 1H), 2.42 (m, 1H), 2.27 (m, 1H), 2.02 (d, 1H), 1.85 (m, 3H), 1.46 (m, 3H).
HPLC-MS: Rt: 3.016 min, m/z: 366.1 (MH⁺).

### Example 26: (1R,4R)-4-(8-methyl-11-oxo-11H-pyrido[2,1-b]quinazoline-6-carboxamido)cyclohexane-1-carboxylic acid

¹H-RMN (400 MHz, DMSO-d₆): δ = 12.12 (s, 1H), 11.05 (d, 1H), 8.80 (s, 1H), 8.46 (d, 1H), 8.30 (d, 1H), 7.95 (m, 2H), 7.76 (d, 1H), 7.55 (t, 1H), 3.82 (m, 1H), 2.39 (s, 3H), 2.33 (m, 1H), 2.09 (m, 2H), 1.99 (m, 2H), 1.49 (m, 4H)
HPLC-MS: Rt: 3.247 min, m/z: 379.8 (MH⁺).

### Example 27: (1S,4S)-4-(2-cyclopentyl-11-oxo-11H-pyrido[2,1-b]quinazoline-6-carboxamido)cyclohexane-1-carboxylic acid

¹H-RMN (400 MHz, DMSO-d₆): δ = 12.25 (s, 1H), 11.32 (d, 1H), 9.00 (dd, 1H), 8.60 (dd, 1H), 8.14 (s, 1H), 7.85 (m, 2H), 7.18 (t, 1H), 4.28 (m, 1H), 3.21 (m, 1H), 2.43 (m, 1H), 2.11 (m, 2H), 1.71 (m, 14H).
HPLC-MS: Rt: 3.661 min, m/z: 433.9 (MH⁺).

### Example 28: (trans)-4-(2-(4-fluorophenyl)-11-oxo-11H-pyrido[2,1-b]quinazoline-6-carboxamido)cyclohexane-1-carboxylic acid

¹H-RMN (400 MHz, DMSO-d₆): δ = 12.13 (s, 1H), 10.99 (d, 1H), 9.00 (dd, 1H), 8.59 (dd, 1H), 8.47 (d, 1H), 8.27 (dd, 1H), 7.86 (m, 3H), 7.36 (m, 2H), 7.20 (t, 1H), 3.83 (m, 1H), 2.34 (m, 1H), 2.09 (m, 2H), 2.00 (m, 2H), 1.52 (m, 4H).
HPLC-MS: Rt: 3.750 min, m/z: 459.8 (MH⁺).

### Example 29: (cis)-4-(2-(3-fluorophenyl)-11-oxo-11H-pyrido[2,1-b]quinazoline-6-carboxamido)cyclohexane-1-carboxylic acid

¹H-RMN (400 MHz, DMSO-d₆): δ = 12.26 (s, 1H), 11.23 (d, 1H), 9.03 (dd, 1H), 8.64 (dd, 1H), 8.55 (d, 1H), 8.27 (dd, 1H), 7.96 (d, 1H), 7.68 (m, 2H), 7.57 (dd, 1H), 7.25 (m, 2H), 4.27 (m, 1H), 2.45 (m, 1H), 1.84 (m, 4H).
HPLC-MS: Rt: 3.647 min, m/z: 459.8 (MH⁺).

### Example 30: (cis)-4-(2-(3,4-difluorophenyl)-11-oxo-11H-pyrido[2,1-b]quinazoline-6-carboxamido)cyclohexane-1-carboxylic acid

¹H-RMN (400 MHz, DMSO-d₆): δ = 12.27 (s, 1H), 11.21 (d, 1H), 9.01 (d, 1H), 8.63 (d, 1H), 8.50 (s, 1H), 8.23 (d, 1H), 7.94 (m, 2H), 7.68 (m, 1H), 7.56 (m, 1H), 7.22 (t, 1H), 4.25 (m, 1H), 2.45 (m, 1H), 1.83 (m, 8H).
HPLC-MS: Rt: 3.728 min, m/z: 477.8 (MH⁺).

### Example 31: (cis)-4-(2-(4-cyanophenyl)-11-oxo-11H-pyrido[2,1-b]quinazoline-6-carboxamido)cyclohexane-1-carboxylic acid

¹H-RMN (400 MHz, DMSO-d₆): δ = 12.28 (s, 1H), 11.20 (d, 1H), 9.04 (d, 1H), 8.65 (d, 1H), 8.59 (d, 1H), 8.30 (dd, 1H), 8.04 (d, 2H), 7.97 (d, 3H), 7.24 (t, 1H), 4.26 (s, 1H), 2.45 (m, 1H), 1.84 (dd, 8H).
HPLC-MS: Rt: 3.506 min, m/z: 466.8 (MH⁺).

### Example 32: (cis)-4-(2-(3-cyanophenyl)-11-oxo-11H-pyrido[2,1-b]quinazoline-6-carboxamido)cyclohexane-1-carboxylic acid

¹H-RMN (400 MHz, DMSO-d₆): δ = 12.27 (s, 1H), 11.22 (d, 1H), 9.04 (dd, 1H), 8.65 (dd, 1H), 8.60 (d, 1H), 8.31 (m, 2H), 8.18 (d, 1H), 7.96 (d, 1H), 7.89 (d, 1H), 7.74 (t, 1H), 7.24 (t, 1H), 4.27 (m, 1H), 2.47 (m, 1H), 1.85 (m, 8H).
HPLC-MS: Rt: 3.476 min, m/z: 466.8 (MH⁺).

### Example 33: 4-(6-(((cis)-4-carboxycyclohexyl)carbamoyl)-11-oxo-11H-pyrido[2,1-b]quinazolin-2-yl)benzoic acid

¹H-RMN (400 MHz, DMSO-d₆): δ = 12.64 (s, 1H), 11.23 (d, 1H), 9.03 (dd, 1H), 8.64 (dd, 1H), 8.58 (d, 1H), 8.28 (dd, 1H), 8.07 (m, 2H), 7.96 (m, 3H), 7.22 (t, 1H), 4.27 (m, 1H), 2.42 (m, 1H), 1.82 (m, 8H).
HPLC-MS: Rt: 2.695 min, m/z: 487.1 (MH⁺).

### Example 34: (cis)-4-(11-oxo-2-(pyridin-3-yl)-11H-pyrido[2,1-b]quinazoline-6-carboxamido)cyclohexane-1-carboxylic acid

¹H-RMN (400 MHz, DMSO-d₆): δ = 12.30 (s, 1H), 11.20 (d, 1H), 9.01 (dd, 2H), 8.63 (dd, 2H), 8.54 (d, 1H), 8.25 (m, 2H), 7.96 (d, 1H), 7.54 (dd, 1H), 7.22 (t, 1H), 4.26 (m, 1H), 2.45 (m, 1H), 1.82 (m, 8H).
HPLC-MS: Rt: 3.105 min, m/z: 442.8 (MH⁺).

### Example 35: (trans)-4-(11-oxo-2-(pyridin-4-yl)-11H-pyrido[2,1-b]quinazoline-6-carboxamido)cyclohexane-1-carboxylic acid

¹H-RMN (400 MHz, DMSO-d₆): δ =10.93 (d, 1H), 9.06 (dd, 1H), 8.81 (m, 2H), 8.78 (d, 1H), 8.65 (dd, 1H), 8.47 (dd, 1H), 8.16 (m, 2H), 7.95 (d, 1H), 7.26 (t, 1H), 3.86 (m, 1H), 2.35 (m, 1H), 2.12 (m, 2H), 2.03 (m, 2H), 1.53 (m, 4H)
HPLC-MS: Rt: 3.272 min, m/z: 443.7 (MH⁺).

### Example 36: (cis)-4-(2-(3-fluoropyridin-4-yl)-11-oxo-11H-pyrido[2,1-b]quinazoline-6-carboxamido)cyclohexane-1-carboxylic acid

¹H-RMN (400 MHz, DMSO-d₆): δ = 11.20 (d, 1H), 9.03 (dd, 1H), 8.72 (d, 1H), 8.67 (dd, 1H), 8.59 (s, 1H), 8.56 (d, 1H), 8.20 (d, 1H), 8.01 (d, 1H), 7.80 (dd, 1H), 7.25 (t, 1H), 4.28 (m, 1H), 2.45 (m, 1H), 1.82 (m, 8H)
HPLC-MS: Rt: 3.204 min, m/z: 461.1 (MH⁺).

### Example 37: (cis)-4-(11-oxo-2-(pyrimidin-5-yl)-11H-pyrido[2,1-b]quinazoline-6-carboxamido)cyclohexane-1-carboxylic acid

¹H-RMN (400 MHz, DMSO-d₆): δ = 12.29 (s, 1H), 11.18 (d, 1H), 9.28 (s, 2H), 9.23 (s, 1H), 9.02 (d, 1H), 8.64 (d, 2H), 8.33 (d, 1H), 7.97 (d, 1H), 7.23 (t, 1H), 4.26 (m, 1H), 2.45 (m, 1H), 1.83 (m, 8H).

### Example 38: (cis)-4-(2-(1-methyl-1H-pyrazol-4-yl)-11-oxo-11H-pyrido[2,1-b]quinazoline-6-carboxamido)cyclohexane-1-carboxylic acid

¹H-RMN (400 MHz, DMSO-d₆): δ = 12.25 (s, 1H), 11.25 (d, 1H), 8.99 (dd, 1H), 8.59 (dd, 1H), 8.40 (d, 1H), 8.35 (s, 1H), 8.13 (dd, 1H), 8.03 (s, 1H), 7.87 (d, 1H), 7.18 (t, 1H), 4.26 (m, 1H), 3.90 (s, 3H), 2.45 (m, 1H), 1.82 (m, 8H).
HPLC-MS: Rt: 3.471 min, m/z: 420.1 (MH⁺).

### Example 39: (cis)-4-(11-oxo-2-(thiophen-2-yl)-11H-pyrido[2,1-b]quinazoline-6-carboxamido)cyclohexane-1-carboxylic acid

¹H-RMN (400 MHz, DMSO-d₆): δ = 12.28 (s, 1H), 11.21 (d, 1H), 9.01 (dd, 1H), 8.62 (dd, 1H), 8.44 (d, 1H), 8.23 (dd, 1H), 7.93 (d, 1H), 7.71 (d, 1H), 7.65 (d, 1H), 7.21 (m, 2H), 4.27 (m, 1H), 2.44 (m, 1H), 1.83 (m, 8H).
HPLC-MS: Rt: 3.561 min, m/z: 448.1 (MH⁺).

### Example 40: (cis)-4-(8-bromo-11-oxo-11H-pyrido[2,1-b]quinazoline-6-carboxamido)cyclohexane-1-carboxylic acid

¹H-RMN (400 MHz, DMSO-d₆): δ = 12.27 (s, 1H), 11.11 (d, 1H), 9.05 (d, 1H), 8.51 (d, 1H), 8.34 (d, 1H), 7.95 (m, 2H), 7.62 (t, 1H), 4.25 (m, 1H), 2.44 (m, 1H), 1.83 (m, 8H)
HPLC-MS: Rt: 3.125 min, m/z: 444.9 (MH⁺).

### Example 41: 3-(2-cyclopropyl-11-oxo-11H-pyrido[2,1-b]quinazoline-6-carboxamido)cyclobutane-1-carboxylic acid

¹H-RMN (400 MHz, DMSO-d₆): δ = 12.24 (s, 1H), 11.17 (d, 1H), 8.95 (dd, 1H), 8.50 (dd, 1H), 7.98 (s, 1H), 7.70 (m, 2H), 7.15 (t, 1H), 4.43 (m, 1H), 2.90 (m, 1H), 2.65 (m, 2H), 2.21 (m, 3H), 1.08 (m, 2H), 0.81 (m, 2H)
HPLC-MS: Rt: 3.190 min, m/z: 377.8 (MH⁺).

### Example 42: (1S,4S)-4-(2-(5-cyanothiophen-2-yl)-11-oxo-11H-pyrido[2,1-b]quinazoline-6-carboxamido)cyclohexane-1-carboxylic acid

¹H-RMN (300 MHz, DMSO-d₆): 12.45 (bs, 1H), 11.16 (d, *J* = 9.0 Hz, 1H), 9.02 (d, *J* = 9.0 Hz, 1H), 8.65 (d, *J* = 6.9 Hz, 1H), 8.51 (s, 1H), 8.24 (d, *J* = 10.9 Hz, 1H), 8.03 (d, *J* = 3.9 Hz, 1H), 7.95 (d, *J* = 8.8 Hz, 1H), 7.87 (d, *J* = 4.0 Hz, 1H), 7.25 (t, *J* = 7.1 Hz, 1H), 4.27 (bs, 1H), 2.42 (bs, 1H), 2.10 - 1.56 (m, 8H).
HPLC-MS: Rt: 5.7 min, m/z: 473.1 (MH⁺).

### Example 43: (1S,4S)-4-(2-(5-carbamoylthiophen-2-yl)-11-oxo-11H-pyrido[2,1-b]quinazoline-6-carboxamido)cyclohexane-1-carboxylic acid

¹H-RMN (400 MHz, DMSO-d₆): 12.40 (bs, 1H), 11.20 (d, *J* = 9.4 Hz, 1H), 9.00 (d, *J* = 7.4 Hz, 1H), 8.62 (d, *J* = 8.6 Hz, 1H), 8.46 (s, 1H), 8.21 (d, *J* = 11.2 Hz, 1H), 8.04 (s, 1H), 7.94 (d, *J* = 8.6 Hz, 1H), 7.75 (d, *J* = 4.0 Hz, 1H), 7.68 (d, *J* = 3.8 Hz, 1H), 7.48 (s, 1H), 7.21 (t, *J* = 7.2 Hz, 1H), 4.25 (bs, 1H), 2.40 (bs, 1H), 1.98 - 1.55 (m, 8H).

### Example 44: (1S,4S)-4-(2-(1-methyl-1H-imidazol-4-yl)-11-oxo-11H-pyrido[2,1-b]quinazoline-6-carboxamido)cyclohexane-1-carboxylic acid

¹H-RMN (300 MHz, DMSO-d₆): 12.33 (bs, 1H), 11.28 (d, *J* = 8.1 Hz, 1H), 9.00 (dd, *J* = 7.3, 1.7 Hz, 1H), 8.60 (dd, *J* = 7.1, 1.7 Hz, 1H), 8.41 (d, *J* = 2.1 Hz, 1H), 8.36 (s, 1H), 8.14 (dd, *J* = 8.6, 2.2 Hz, 1H), 8.03 (s, 1H), 7.88 (d, *J* = 8.6 Hz, 1H), 7.18 (t, *J* = 7.1 Hz, 1H), 4.26 (bs, 1H), 3.90 (s, 3H), 3.17 (s, 1H), 1.96 - 1.63 (m, 6H), 1.20 (m, 2H).
HPLC-MS: Rt: 8.45 min, m/z: 446.1 (MH⁺).

### Example 45: (1S,4S)-4-(2-(5-cyclopropylthiophen-2-yl)-11-oxo-11H-pyrido[2,1-b]quinazoline-6-carboxamido)cyclohexane-1-carboxylic acid

¹H-RMN (300 MHz, DMSO-d₆): 13.54 (bs, 1H), 11.26 (d, *J* = 9.8 Hz, 1H), 9.04 - 8.88 (m, 1H), 8.58 (d, *J* = 8.3 Hz, 1H), 8.15 (s, 1H), 8.03 (d, *J* = 8.3 Hz, 1H), 7.90 (d, *J* = 9.2 Hz, 1H), 7.31 (s, 1H), 7.18 (t, *J* = 7.3 Hz, 1H), 6.71 (s, 1H), 4.28 (s, 1H), 2.35 (s, 1H), 2.10 - 2.01 (m, 1H), 1.86 (s, 4H), 1.75 (s, 4H), 1.10 - 0.91 (m, 2H), 0.76 - 0.62 (m, 2H).
HPLC-MS: Rt: 17.10 min, m/z: 487,57 (MH⁺).

### Example 46: (1S,4S)-4-(11-oxo-2-(thiazol-5-yl)-11H-pyrido[2,1-b]quinazoline-6-carboxamido)cyclohexane-1-carboxylic acid

¹H-RMN (300 MHz, DMSO-d₆): 12.38 (bs, 1H), 11.18 (d, *J* = 8.2 Hz, 1H), 9.14 (s, 1H), 9.00 (d, *J* = 8.2 Hz, 1H), 8.62 (d, *J* = 7.0 Hz, 1H), 8.49 (s, 1H), 8.43 (s, 1H), 8.23 (d, *J* = 11.3 Hz, 1H), 7.94 (d, *J* = 8.6 Hz, 1H), 7.22 (t, *J* = 6.9 Hz, 1H), 4.26 (s, 1H), 2.41 (s, 1H), 2.06 - 1.81 (m, 4H), 1.82 - 1.44 (m, 4H).

### Example 47: 5-(6-(((1s,4s)-4-carboxycyclohexyl)carbamoyl)-11-oxo-11H-pyrido[2,1-b]quinazolin-2-yl)thiophene-2-carboxylic acid

¹H-RMN (300 MHz, DMSO-d₆): 12.65 (bs, 2H), 11.21 (d, *J* = 9.2 Hz, 1H), 8.99 (d, *J* = 7.0 Hz, 1H), 8.61 (d, *J* = 9.2 Hz, 1H), 8.43 (s, 1H), 8.22 (d, *J* = 11.4 Hz, 1H), 7.91 (d, *J* = 11.4 Hz, 1H), 7.59 (d, *J* = 3.3 Hz, 1H), 7.46 (d, *J* = 3.3 Hz, 1H), 7.27 - 7.13 (m, 1H), 4.24 (bs, 1H), 2.40 (bs, 1H), 1.95 - 1.62 (m, 6H), 1.29 - 1.06 (m, 2H).

## Claims

1. A compound of formula (I): 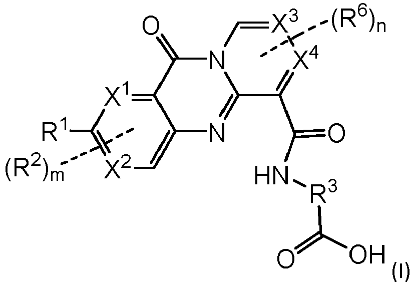 wherein:
- R¹ represents a group selected from:
a) C₃-C₆ cycloalkyl optionally substituted by linear or branched C₁-C₆ alkyl, linear orbranched C₁-C₄ alkoxy and halogen atom,
b) C₃-C₆ heterocyclic ring containing 1 or 2 heteroatoms selected from N, O and S and which is optionally substituted by halogen atom, linear or branched C₁-C₆ haloalkyl, linear or branched C₁-C₆ alkyl, C₃-C₄ cycloalkyl, cyano group, -COOH, -CONH₂, SO₂NR₄R₅, -SO₂CH₃, NH₂, -NHC(O)R₄ and linear or branched C₁-C₄ alkoxy,
c) halogen atom,
d) hydrogen atom,
e) cyano group,
f) C₁-C₃ alkoxy optionally substituted by 1, 2 or 3 halogen atoms,
g) -OH,
h) phenyl ring optionally substituted by a group selected from halogen atom, linear or branched C₁-C₆ haloalkyl, linear or branched C₁-C₆ alkyl, C₃-C₄ cycloalkyl, cyano group, -COOH, -CONH₂, SO₂NR₄R₅, -SO₂CH₃, NH₂, -NHC(O)R₄ and linear or branched C₁-C₄ alkoxy,
i) -S(O)ₚR⁷, wherein R⁷ is C₁-C₃ alkyl and p is an integer selected from 1 to 2, and
j) linear or branched C₁-C₆ alkyl optionally substituted by 1, 2 or 3 halogen atoms
- each one of R² and R⁶ independently represents a group selected from:
a) halogen atom,
b) linear or branched C₁-C₆ alkyl,
c) linear or branched C₁-C₆ haloalkyl, and
d) phenyl or C₄-C₆ heterocyclic ring containing 1 or 2 heteroatoms selected from N, O and S and which are optionally substituted by a group selected from halogen atom, linear or branched C₁-C₆ haloalkyl, linear or branched C₁-C₆ alkyl and linear or branched C₁-C₄ alkoxy,
e) C₃-C₆ cycloalkyl optionally substituted by linear or branched C₁-C₆ alkyl and linear or branched C₁-C₄ alkoxy,
wherein the group R², if present, replaces the hydrogen atom of one of the groups CH-of X¹ and X² and R⁶, if present, replaces the hydrogen atom of one of the groups CH- of X³ and X⁴,
- m and n are integers independently selected from 0 and 1,
- R³ represents a group selected from:
a) 4- to 10- membered, saturated cycle optionally containing 1 or 2 heteroatoms selected from N and O, which is optionally substituted by 1, 2 or 3 groups selected from linear or branched C₁-C₆ alkyl, linear or branched C₁-C₆ alkoxy, -OH and-NR⁴R⁵,
b) linear or branched C₁-C₆ alkyl,
- R⁴ and R⁵ represent independently a group selected from hydrogen atom, linear or branched C₁-C₆ alkyl and C₃-C₆ cycloalkyl group,
- X¹ and X² are independently selected from CH and N with the condition that either none or one of them is N,
- X³ and X⁴ are independently selected from CH and N with the condition that either none or one of them is N,
and pharmaceutically acceptable salts thereof.

2. A compound according to claim 1 wherein R¹ represents a group selected from:
- C₃-C₆ cycloalkyl optionally substituted by linear or branched C₁-C₆ alkyl and linear or branched C₁-C₄ alkoxy,
- C₄-C₆ heterocyclic ring containing 1 or 2 heteroatoms selected from N and O and which is optionally substituted by linear or branched C₁-C₆ alkyl,
- phenyl ring optionally substituted by a group selected from halogen atom, linear or branched C₁-C₆ haloalkyl, linear or branched C₁-C₆ alkyl and linear or branched C₁-C₄ alkoxy.

3. A compound according to claim 2 wherein R¹ represents a cyclopropyl group optionally substituted by a group selected from linear or branched C₁-C₆ alkyl and linear or branched C₁-C₄ alkoxy.

4. A compound according to claim 2 wherein R¹ represents a group selected from pyridinyl, piperazinyl and morpholinyl group optionally substituted by a linear or branched C₁-C₆ alkyl group.

5. A compound according to claim 2 wherein R¹ represents a phenyl ring optionally substituted by a group selected from halogen atom and linear or branched C₁-C₆ haloalkyl.

6. A compound according to any one of claims 1 to 5 wherein each one of m and n have a value of 0.

7. A compound according to any one claims 1 to 6 wherein R³ represents a group selected from cyclopentyl and cyclohexyl group optionally substituted by a group selected from linear or branched C₁-C₆ alkyl and -OH.

8. A compound according to any one claims 1 to 7 wherein X¹, X², X³ and X⁴ represent CH.

9. A compound according to claim 1 wherein R¹ represents a cyclopropyl group optionally substituted by a group selected from linear or branched C₁-C₆ alkyl and linear or branched C₁-C₄ alkoxy, both m and n have a value of 0, R³ represents a cyclohexyl group optionally substituted by a group selected from linear or branched C₁-C₆ alkyl and -OH and X¹, X², X³ and X⁴ represent CH.

10. A compound according to claim 1 wherein R¹ represent a group selected from:
- pyridinyl, piperazinyl, and morpholinyl group optionally substituted by linear or branched C₁-C₆ alkyl, or
- phenyl ring optionally substituted by a group selected from halogen atom and linear or branched C₁-C₆ haloalkyl,
both m and n have a value of 0, R³ represents a cyclohexyl group optionally substituted by a group selected from linear or branched C₁-C₆ alkyl and -OH and X¹, X², X³ and X⁴ represent CH.

11. A compound according to claim 1 which is one of:
4-(2-cyclopropyl-11-oxo-11H-pyrido[2,1-b]quinazoline-6-carboxamido)cyclohexane-1-carboxylic acid
(1s,4s)-4-(2-(5-methylthiophen-2-yl)-11-oxo-11H-pyrido[2,1-b]quinazoline-6-carboxamido)cyclohexane-1-carboxylic acid
(1s,4s)-4-(2-cyclopropyl-11-oxo-11H-pyrido[2,1-b]quinazoline-6-carboxamido)cyclohexane-1-carboxylic acid
(1r,4r)-4-(2-cyclopropyl-11-oxo-11H-pyrido[2,1-b]quinazoline-6-carboxamido)cyclohexane-1-carboxylic acid
(1s,4s)-4-(11-oxo-2-(trifluoromethoxy)-11H-pyrido[2,1-b]quinazoline-6-carboxamido)cyclohexane-1-carboxylic acid
4-(2-cyclopropyl-11-oxo-11H-pyrido[2,1-b]quinazoline-6-carboxamido)bicyclo[2.2.2]octane-1-carboxylic acid
4-(2-cyclopropyl-11-oxo-11H-pyrido[2,1-b]quinazoline-6-carboxamido)bicyclo[2.2.1]heptane-1-carboxylic acid
1-(2-cyclopropyl-11-oxo-11H-pyrido[2,1-b]quinazoline-6-carboxamido)cyclopentane-1-carboxylic acid
2-(2-cyclopropyl-11-oxo-11H-pyrido[2,1-b]quinazoline-6-carboxamido)-2-methylpropanoic acid
4-(8-methyl-11-oxo-11H-pyrido[2,1-b]quinazoline-6-carboxamido)cyclohexane-1-carboxylic acid
(1s,4s)-4-(8-methyl-11-oxo-11H-pyrido[2,1-b]quinazoline-6-carboxamido)cyclohexane-1-carboxylic acid
4-(11-oxo-11H-pyrido[2,1-b]quinazoline-6-carboxamido)cyclohexane-1-carboxylic acid
(1s,4s)-4-(11-oxo-11H-pyrido[2,1-b]quinazoline-6-carboxamido)cyclohexane-1-carboxylic acid
(1s,4s)-4-(2-morpholino-11-oxo-11H-pyrido[2,1-b]quinazoline-6-carboxamido)cyclohexane-1-carboxylic acid
(1s,4s)-4-(11-oxo-2-phenyl-11H-pyrido[2,1-b]quinazoline-6-carboxamido)cyclohexane-1-carboxylic acid
(1s,4s)-4-(2-(4-fluorophenyl)-11-oxo-11H-pyrido[2,1-b]quinazoline-6-carboxamido)cyclohexane-1-carboxylic acid
(1s,4s)-4-(11-oxo-2-(pyridin-4-yl)-11H-pyrido[2,1-b]quinazoline-6-carboxamido)cyclohexane-1-carboxylic acid
(1s,4s)-4-(2-cyclopropyl-8-methyl-11-oxo-11H-pyrido[2,1-b]quinazoline-6-carboxamido)cyclohexane-1-carboxylic acid
4-(2-hydroxy-11-oxo-11H-pyrido[2,1-b]quinazoline-6-carboxamido)cyclohexane-1-carboxylic acid
4-(2-methoxy-11-oxo-11H-pyrido[2,1-b]quinazoline-6-carboxamido)cyclohexane-1-carboxylic acid
4-(2-cyano-11-oxo-11H-pyrido[2,1-b]quinazoline-6-carboxamido)cyclohexane-1-carboxylic acid
4-(2-fluoro-11-oxo-11H-pyrido[2,1-b]quinazoline-6-carboxamido)cyclohexane-1-carboxylic acid
4-(2-chloro-11-oxo-11H-pyrido[2,1-b]quinazoline-6-carboxamido)cyclohexane-1-carboxylic acid
4-(2-bromo-11-oxo-11H-pyrido[2,1-b]quinazoline-6-carboxamido)cyclohexane-1-carboxylic acid
3-(11-oxo-11H-pyrido[2,1-b]quinazoline-6-carboxamido)cyclohexane-1-carboxylic acid
(1r,4r)-4-(8-methyl-11-oxo-11H-pyrido[2,1-b]quinazoline-6-carboxamido)cyclohexane-1-carboxylic acid
(1s,4s)-4-(2-cyclopentyl-11-oxo-11H-pyrido[2,1-b]quinazoline-6-carboxamido)cyclohexane-1-carboxylic acid
(trans)-4-(2-(4-fluorophenyl)-11-oxo-11H-pyrido[2,1-b]quinazoline-6-carboxamido)cyclohexane-1-carboxylic acid
(cis)-4-(2-(3-fluorophenyl)-11-oxo-11H-pyrido[2,1-b]quinazoline-6-carboxamido)cyclohexane-1-carboxylic acid
(cis)-4-(2-(3,4-difluorophenyl)-11-oxo-11H-pyrido[2,1-b]quinazoline-6-carboxamido)cyclohexane-1-carboxylic acid
(cis)-4-(2-(4-cyanophenyl)-11-oxo-11H-pyrido[2,1-b]quinazoline-6-carboxamido)cyclohexane-1-carboxylic acid
(cis)-4-(2-(3-cyanophenyl)-11-oxo-11H-pyrido[2,1-b]quinazoline-6-carboxamido)cyclohexane-1-carboxylic acid
4-(6-(((cis)-4-carboxycyclohexyl)carbamoyl)-11-oxo-11H-pyrido[2,1-b]quinazolin-2-yl)benzoic acid
(cis)-4-(11-oxo-2-(pyridin-3-yl)-11H-pyrido[2,1-b]quinazoline-6-carboxamido)cyclohexane-1-carboxylic acid
(trans)-4-(11-oxo-2-(pyridin-4-yl)-11H-pyrido[2,1-b]quinazoline-6-carboxamido)cyclohexane-1-carboxylic acid
(cis)-4-(2-(3-fluoropyridin-4-yl)-11-oxo-11H-pyrido[2,1-b]quinazoline-6-carboxamido)cyclohexane-1-carboxylic acid
(cis)-4-(11-oxo-2-(pyrimidin-5-yl)-11H-pyrido[2,1-b]quinazoline-6-carboxamido)cyclohexane-1-carboxylic acid
(cis)-4-(2-(1-methyl-1H-pyrazol-4-yl)-11-oxo-11H-pyrido[2,1-b]quinazoline-6-carboxamido)cyclohexane-1-carboxylic acid
(cis)-4-(11-oxo-2-(thiophen-2-yl)-11H-pyrido[2,1-b]quinazoline-6-carboxamido)cyclohexane-1-carboxylic acid
(cis)-4-(8-bromo-11-oxo-11H-pyrido[2,1-b]quinazoline-6-carboxamido)cyclohexane-1-carboxylic acid
3-(2-cyclopropyl-11-oxo-11H-pyrido[2,1-b]quinazoline-6-carboxamido)cyclobutane-1-carboxylic acid
(1s,4s)-4-(2-(5-cyanothiophen-2-yl)-11-oxo-11H-pyrido[2,1-b]quinazoline-6-carboxamido)cyclohexane-1-carboxylic acid
(1s,4s)-4-(2-(5-carbamoylthiophen-2-yl)-11-oxo-11H-pyrido[2,1-b]quinazoline-6-carboxamido)cyclohexane-1-carboxylic acid
(1s,4s)-4-(2-(1-methyl-1H-imidazol-4-yl)-11-oxo-11H-pyrido[2,1-b]quinazoline-6-carboxamido)cyclohexane-1-carboxylic acid
(1s,4s)-4-(2-(5-cyclopropylthiophen-2-yl)-11-oxo-11H-pyrido[2,1-b]quinazoline-6-carboxamido)cyclohexane-1-carboxylic acid
(1s,4s)-4-(11-oxo-2-(thiazol-5-yl)-11H-pyrido[2,1-b]quinazoline-6-carboxamido)cyclohexane-1-carboxylic acid
5-(6-(((1s,4s)-4-carboxycyclohexyl)carbamoyl)-11-oxo-11H-pyrido[2,1-b]quinazolin-2-yl)thiophene-2-carboxylic acid
and pharmaceutically aceptable salts thereof.

12. Use of compound according to any one claims 1 to 11 in the manufacture of a medicament for the treatment of a disease or pathological condition wherein diseases or pathological condition is selected from the group consisting of liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric, lung, pancreatic, breast, colon, colorectal and other diseases selected from asthma, chronic obstructive pulmonary disease (COPD), transplant rejection, haematological disease, uveitis, dry eye, allergic conjunctivitis and neurodegenerative diseases including Alzheimer disease.

13. Compound of formula (I) as defined in any one claims 1 to 11 for use in the treatment of a disease or pathological condition wherein diseases or pathological condition is selected from the group consisting of liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric, lung, pancreatic, breast, colon, colorectal and other diseases selected from asthma, chronic obstructive pulmonary disease (COPD), transplant rejection, haematological disease, uveitis, dry eye, allergic conjunctivitis and neurodegenerative diseases including Alzheimer disease.

14. A pharmaceutical composition comprising a compound as defined in any one of claims 1 to 11 and a pharmaceutically acceptable diluent or carrier.

15. A pharmaceutical composition according to claim 14 further comprising a therapeutically effective amount of a therapeutic agent selected from agent useful for the treatment of liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric, lung, pancreatic, breast, colon, colorectal and other diseases selected from asthma, chronic obstructive pulmonary disease (COPD), transplant rejection, haematological disease, uveitis, dry eye, allergic conjunctivitis and neurodegenerative diseases including Alzheimer disease.

16. A combination product comprising a compound according to any one claims 1 to 11 and at least a therapeutic agent selected from an agent useful for the treatment of liver diseases including non-alcoholic steatohepatitis (NASH) and cirrhosis of the liver, autoimmune diseases including psoriasis, atopic dermatitis, rheumatoid arthritis, multiple sclerosis, alopecia areata, inflammatory bowel diseases including ulcerative colitis and Crohn's disease, cancer including gastric, lung, pancreatic, breast, colon, colorectal, and others diseases selected from asthma, chronic obstructive pulmonary disease (COPD), transplant rejection, haematological disease, uveitis, dry eye, allergic conjunctivitis and neurodegenerative diseases including Alzheimer disease.


SEQUENCE LISTING

<110>  ONCOSTELLAE, S.L.

<120>  CARBOXILIC ACID DERIVATIVES AS PROTEIN KINASES INHIBITORS

<130>  P14758PC00

<150>  ES P201730760
<151>  2017-06-01

<160>  2

<170>  PatentIn version 3.5

<210>  1
<211>  13
<212>  PRT
<213>  Artificial Sequence

<220>
<223>  Peptide

<400>  1

Lys Lys Ser Arg Gly Asp Tyr Met Thr Met Gln Ile Gly
1               5                   10

<210>  2
<211>  13
<212>  PRT
<213>  Artificial Sequence

<220>
<223>  Peptide

<400>  2

Glu Glu Pro Leu Tyr Trp Ser Phe Pro Ala Lys Lys Lys
1               5                   10